(19) Europäisches Patentamt
European Patent Office
Office européen des brevets

(11) EP 2 935 187 B1

(12) EUROPÄISCHE PATENTSCHRIFT

(45) Veröffentlichungstag und Bekanntmachung des Hinweises auf die Patenterteilung:
**23.11.2016 Patentblatt 2016/47**

(21) Anmeldenummer: **13802052.4**

(22) Anmeldetag: **09.12.2013**

(51) Int Cl.:
***C07C 45/50*** *(2006.01)*

(86) Internationale Anmeldenummer:
**PCT/EP2013/075933**

(87) Internationale Veröffentlichungsnummer:
**WO 2014/095452 (26.06.2014 Gazette 2014/26)**

# (54) STEUERUNG DER VISKOSITÄT VON REAKTIONSLÖSUNGEN IN HYDROFORMYLIERUNGVERFAHREN

CONTROL OF THE VISCOSITY OF REACTION SOLUTIONS IN HYDROFORMYLATION METHODS

COMMANDE DE LA VISCOSITÉ DE SOLUTIONS DE RÉACTION DANS DES PROCÉDÉS D'HYDROFORMYLATION

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **18.12.2012 DE 102012223572**

(43) Veröffentlichungstag der Anmeldung:
**28.10.2015 Patentblatt 2015/44**

(73) Patentinhaber: **Evonik Degussa GmbH**
**45128 Essen (DE)**

(72) Erfinder:
- **FRIDAG, Dirk**
  **45721 Haltern am See (DE)**
- **LENZ,Udo**
  **45657 Recklinghausen (DE)**
- **LUEKEN, Hans-Gerd**
  **45770 Marl (DE)**
- **FRANKE, Robert**
  **45772 Marl (DE)**
- **RUDEK, Markus**
  **53123 Bonn (DE)**
- **WIESE, Klaus-Diether**
  **45721 Haltern am See (DE)**
- **BRÖCKER, Sönke**
  **64372 Ober-Ramstadt (DE)**
- **PRISKE, Markus**
  **Mobile, AL 36609 (US)**
- **PATALONG, Christoph**
  **44575 Castrop-Rauxel (DE)**

(56) Entgegenhaltungen:
**EP-A2- 0 823 282 EP-B1- 1 931 472**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die vorliegende Erfindung betrifft ein Verfahren zur Steuerung der Viskosität von Reaktionslösungen in Hydroformylierungverfahren in denen katalytisch aktive Zusammensetzungen auf Basis von Übergangsmetallkomplexkatalysatoren, insbesondere Rhodiumkomplexkatalysatoren, in gelöster Form vorliegen.

Die Reaktionen zwischen Olefinverbindungen, Kohlenmonoxid und Wasserstoff in Gegenwart eines Katalysators zu den um ein C-Atom reicheren Aldehyden ist als Hydroformylierung bzw. Oxierung bekannt. In den katalytisch aktiven Zusammensetzungen werden als Übergangsmetallkomplexkatalysatoren häufig in diesen Reaktionen Verbindungen der Übergangsmetalle der VIII. Gruppe des Periodensystems der Elemente verwendet.

Bekannte Liganden in diesen katalytisch aktiven Zusammensetzungen sind beispielsweise Verbindungen aus den Klassen der Phosphine, Phosphite, Phosphinite, Phosphoramidite und Phosphonite mit jeweils dreiwertigen Phosphor $P^{III}$.

Eine gute Übersicht über den Stand der Hydroformylierung von Olefinen findet sich in B. CORNILS, W. A. HERRMANN, "Applied Homogeneous Catalysis with Organometallic Compounds", Vol. 1 & 2, VCH, Weinheim, New York, 1996 bzw. R. Franke, D. Selent, A. Börner, "Applied Hydroformylation", Chem. Rev., 2012, DOI: 10.1021/cr3001803.

Eine klassische Anwendung ist die Erzeugung von C5 Aldehyden durch Hydroformylierung von ungesättigten C4-Verbindungen, die in petrochemischen Verarbeitungsanlagen anfallen. Entsprechend industriell verfügbare Ausgangsstoffe sind Kohlenwasserstoffgemische, die 1-Buten, (Z- und E-) 2-Buten, Isobuten und mehrfach ungesättigte und gesättigte Kohlenwasserstoffe enthalten.

Die katalysierte Hydroformylierung von Olefinen zu den entsprechenden Aldehyden erfolgt üblicherweise in homogener, flüssiger Phase, d.h. Olefin und Produkte liegen in einer Phase vor, wobei die katalytisch aktive Zusammensetzung homogen im flüssigen Reaktionsgemisch gelöst ist.
Zusätzlich kann im Reaktionsgemisch ein inertes Lösungsmittel für die katalytisch aktive Zusammensetzung vorhanden sein.

Als Produkte der Hydroformylierung werden neben dem besagten Aldehyd als primärem Produkt typischerweise auch höher siedende Folgeprodukte (üblicherweise als Hochsieder bezeichnet) gebildet; siehe in Rhodium-catalyzed Hydroformylation, ed. by P.W.N.M. van Leeuwen et C. Claver, Kluwer Academic Publishers 2006, AA Dordrecht, NL, Seite 206-211. Hier wird neben der Hochsiederbildung auch die Abtrennung der Hochsieder und Katalysatorgifte über Destillation von der Katalysatorlösung beschrieben. Als alternativer Weg wird hier auch die Verbrennung der Rhodiumhaitigen Rückstände angeführt.

Unter "Hochsiedern" werden hier Stoffe verstanden, die bei einer höheren Temperatur sieden und höhere Molmassen aufweisen als das primäre Hydroformylierungsprodukt (Aldehyd mit einem Kohlenstoff-Atom mehr als das eingesetzte Olefin) und der daraus durch Hydrierung erhaltene Alkohol. Hochsieder entstehen durch Folgereaktionen aus dem primären Hydroformylierungsprodukt. Zu den typischerweise bei technischen Hydroformylierungen entstehenden Hochsiedern gehören Aldolisierungsprodukte und Acetalisierungsprodukte sowie Ester, die durch Reaktion von Alkoholen und Säuren entstehen, wobei die Alkohole und Säuren insbesondere durch Disproportionierung von Aldehyden gebildet werden.

Bei der technischen Hydroformylierung entsteht typischerweise ein Produktgemisch, das neben dem primären Produkt n-Aldehyd, Folgeprodukte in Form von Hochsiedern und den Übergangsmetallkomplex-Katalysator und dessen freie Liganden umfasst. Je nach Umsatzleistung der Reaktion kann das aus dem Reaktor entnommene Produktgemisch auch nicht umgesetztes Edukt enthalten, also Olefin, Wasserstoff oder Kohlenmonoxid.
Um die Reinheit des primären Produkts zu erhöhen und die katalytisch aktive Zusammensetzung zurückzugewinnen, ist es erforderlich, die Bestandteile Aldehyd, Folgeprodukte und Übergangsmetallkomplex-Katalysator und der gegebenenfalls nicht umgesetzten Einsatzstoffe des bei der Hydroformylierung erhaltenen Produktgemisches voneinander zu trennen.

In DE 10 2008 002 187 A1 wird eine Rhodium-haltige katalytisch aktive Zusammensetzung zur Hydroformylierung von C4 Strömen mit einem Bisphosphitliganden unter Zusatz eines Stabilisators beschrieben. Das Produkt wird durch einen klassischen Strippgasstrom aus der Reaktionslösung entfernt und anschließend auskondensiert.

In dieser Schrift wird auch über die Bildung unlöslicher Folgeprodukte des Liganden berichtet, die über eine Filtration aus der Reaktionslösung entfernt werden.

In WO 2010/003073 wird eine Abtrennung der Hochsieder aus einem Hydroformylierungsprozess über ein Gas-Strippverfahren beschrieben, welches so betrieben wird, dass die Reaktionsmischung aus der Reaktion in eine thermische Aufarbeitung gefahren wird, die unter Zuhilfenahme eines Strippgases neben den Produkten auch die Hochsieder anteilig aus der Reaktionsmischung zu entfernen.
Dieses Verfahren ist nur in der Lage Komponenten über ihren Siedepunkt aus dem System zu entfernen.

In EP 1931472 B1 wird die Verwendung einer Membranfiltration zur Abtrennung von Rhodiumkomplexkatalysatoren vor einer thermischen Aufarbeitung eingesetzt. Der Hochsiederstrom aus dieser thermischen Abtrennung wird ebenfalls noch einmal mit Hilfe einer Membran aufgearbeitet und ein Hochsiederstrom ausgeschleust. Der Retentatstrom

der zweiten Membrananlage wird wieder der Reaktion zugeführt.

In EP 1 232 008 wird vor der Membranfiltration das Reaktionsgemisch entspannt und mit einem Verdünnungsmittel versetzt um ein verblocken der Membran zu verhindern.

In EP1931472B1 wird die organophilen Nanofiltration zur Abscheidung homogen gelösten Katalysatorkomplexen aus Hydroformylierungsmischungen beschrieben.

Die in der Hydroformylierung eingesetzte katalytisch aktive Zusammensetzung, insbesondere die Phosphitliganden können auf verschiedenen Wegen abgebaut werden.

In US 5364950, wie auch in US 5763677 und in "Catalyst Separation, Recovery and Recycling", herausgegeben v. D.J. Cole-Hamilton, R.P. Tooze, 2006, NL, Seiten 25-26, wird die Bildung von sogenannten "*Poisoning Phosphites"* als Neben- bzw. Ligandenabbaureaktion beschrieben. Diese "*Poisoning Phosphites"* bilden sich bei der Verwendung von arylphosphit-modifizierten Rhodium-Komplexen während der Hydroformylierungsreaktion. Hierbei kommt es im Zuge des Abbaus der Liganden in der katalytisch aktiven Zusammensetzung zu einem Austausch einer Arylgruppe durch eine Alkylgruppe des Hydroformylierungsproduktes.

Die Phosphitliganden können auch im Zuge einer Hydrolysereaktion durch die bei der Aldehydkondensation (Hochsiederbildung) gebildeten Wasserspuren abgebaut werden, siehe Paul C.J. Kamer, Joost N.H.Reek und Piet W.N.M van Leeuwen berichten in "Rhodium Catalysed Hydroformylation" Volume 22, Seite 44. Eine Konsequenz aus diesen Abbaureaktionen der Liganden ist, dass die Konzentration an hydroformylierungsaktiven Rhodiumkomplexspezies im Laufe der Zeit abnimmt und mit einem Verlust an Reaktivität einher geht. Es ist allgemein bekannt, dass bei einer kontinuierlichen Fahrweise der Hydroformylierung Ligand/en und - gegebenenfalls weitere Komponenten - während des Reaktionsverlaufs nachdosiert, d. h. nach Reaktionsbeginn zusätzlich zugegeben werden müssen (siehe DE 10 2008 002 187 A1).

Die Stabilität kann zwar durch entsprechende Modifikation des Liganden verbessert, ein Abbau der in der Hydroformylierung katalytisch aktiven Zusammensetzung, insbesondere der verwendeten Liganden, aber nicht gänzlich verhindert werden. Im Fortgang dieser Anmeldung werden die Begriffe Ligandenabbau sowie Ligandenabbauprodukte gleich bedeutend mit Abbau der in der Hydroformylierung katalytisch aktiven Zusammensetzung wie auch Abbauprodukte der in der Hydroformylierung katalytisch aktiven Zusammensetzung verwendet.

Auch D.R. Bryant berichtet in "Catalyst Separation, Recovery and Recycling" Springer 2006 im Kapitel 2 2.6.1.6 bis 2.6.1.8 von der Hydrolyse der Phosphite über verschiedene Zwischenstufen.

Die folgende Darstellung zeigt schematisch die entstehenden Abbauprodukte aus dem Liganden, Kurzname Biphephos oder (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine). Zwischenstufen sind nicht mit in dieser Darstellung mit aufgenommen und es wird von einem hydrolytischen Abbau ausgegangen.

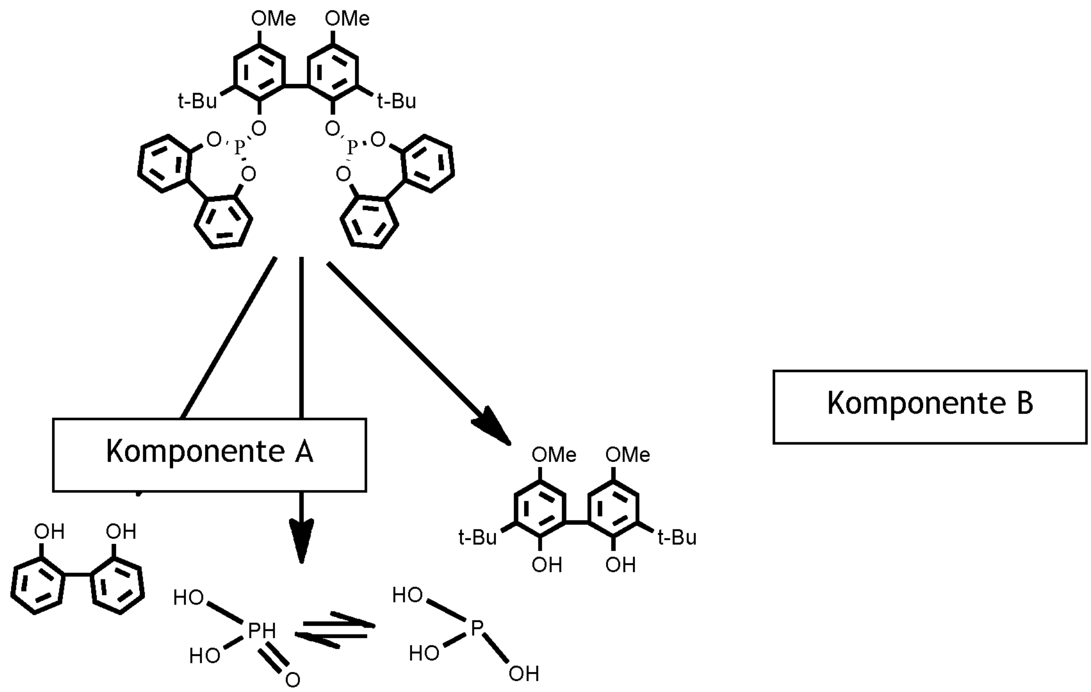


Analytisch konnten die Komponenten A - 2, 2'-Biphenol - und B -3,3'-di-tert-butyl-5,5'-dimethoxy-(1,1'-biphenyl)-2,2'-diol- nachgewiesen werden (NMR).

Die Hydroylse der Phosphitliganden wird ebenfalls in "Homogeneous Catalysts: Activity - Stability - Deactivation, First Edition". Piet W.N.M. van Leeuwen und John C. Chadwick. beschrieben.

In den Schriften werden beispielsweise folgende Phosphite aufgeführt, die chemisch einem gleichen Abbaumechanismus unterliegen und somit zu entsprechenden Alkoholen, Diolen und gegebenenfalls Carbonsäurederivaten, insbesondere Phenolen, Biphenolen, Binaphtholen führen.

Ligand 5b

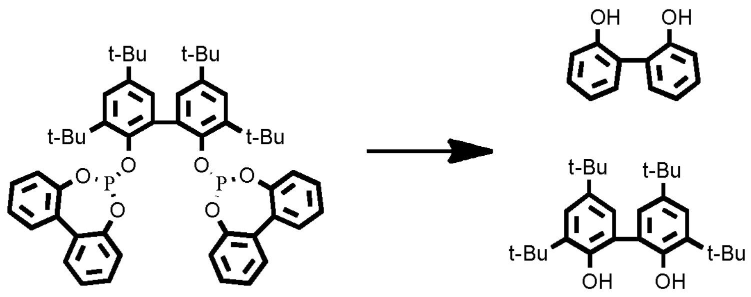

Ligand nach Gladfelter ("Rhodium Catalysed Hydroformylation") Seite 54

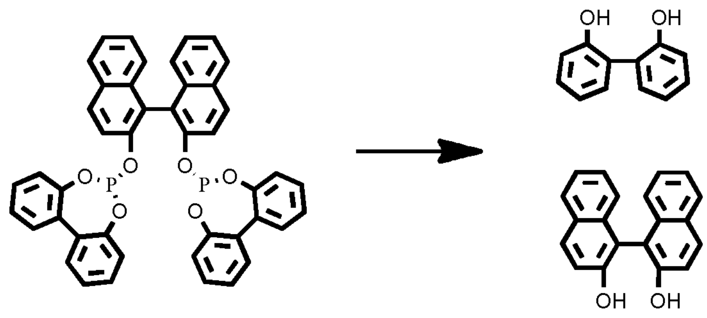

Ligand Mitsubishi("Rhodium Catalysed Hydroformylation") Seite 58

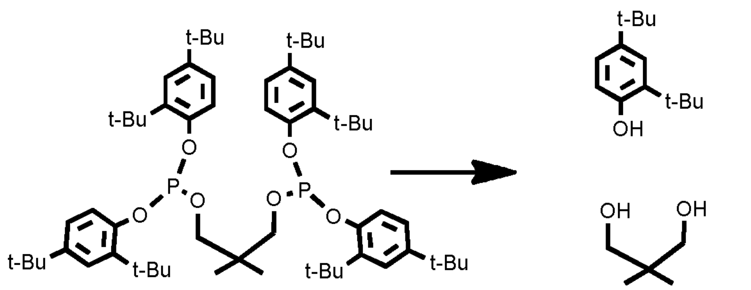

Diese verschiedenen Ligandenbruchstücke und Ligandenabbauprodukte können mit den bisher im Stand der Technik beschriebenen Verfahren nicht abgetrennt werden. Dadurch kommt es zu einer Akkumulation dieser Abbauprodukte.

Diese Akkumulation der Abbauprodukte führt zu einem deutlichen Ansteigen der Viskosität in der Reaktionslösung. Ein solcher Viskositätsanstieg führt in der Regel zu Stoffübergangsproblemen, die wiederum zu einem Abfall der Gesamtaktivität des Prozesses führen.
Der in DE 10 2008 002 187 A1 beschriebene Feststoffanfall führt zu einem erhöhten Aufwand, da diese Filter in regelmäßigen Abständen gereinigt und ausgetauscht werden müssen.

Ausgehend von diesem Stand der Technik bestand die Aufgabe der vorliegenden Erfindung darin, die Viskosität der Reaktionslösung über lange Zeiträume konstant zu halten, um somit einen Feststoffanfall sowie einen Reaktivitätsabfall zu vermeiden.
Überraschenderweise wurde gefunden, dass bei einer Abtrennung der Reaktionsprodukte über die Gasphase mittels ihrer jeweiligen Partialdrücke durch die Einleitung eines "Strip-Gases" und einer Ausschleusung der entstehenden Ligandenabbauprodukte aus den eingesetzten Liganden sowie der Rückführung der in der Hydroformylierung aktiven

katalytischen Zusammensetzung mittels Membranfiltration die Viskosität der Reaktionslösung über lange Zeiträume konstant gehalten werden kann. Der Anfall von Feststoff, wie er in DE 10 2008 002 187 A1 beschrieben wird, tritt nicht mehr auf. Gegenstände der vorliegenden Erfindung sind:

1.) ein Verfahren zur Steuerung der Viskosität von Reaktionslösungen in der Hydroformylierung von olefinhaltigen Gemischen umfassend die Schritte:

i) Bereitstellen eines Gemisches enthaltend gesättigte und olefinsch ungesättigte Kohlenwasserstoffe, einer in der Hydroformylierung katalytisch aktiven Zusammensetzung, eines Gasgemisches aus Kohlenmonoxid und Wasserstoff und mindestens eines Lösungsmittels;
ii) Inkontaktbringen der Edukte in mindestens einer Reaktionszone.;
iii) Abtrennen der Produkte, wobei ein Gasstrom, enthaltend ein Gemisch aus gesättigten und olefinisch ungesättigten Kohlenwasserstoffen, Kohlenmonoxid sowie Wasserstoff in mindestens eine Reaktionszone eingeleitet wird unter der Maßgabe, dass die Produkte über die Gasphase aus der Reaktionszone geführt werden;
iv) Kondensieren der über die Gasphase abgetrennten Produkte und zuführen zur weiteren Aufarbeitung;
v) Leiten des Sumpfstroms aus mindestens einer Reaktionszone auf mindestens eine Membranfiltration, dadurch gekennzeichnet, dass die in der Hydroformylierung katalytisch aktive Zusammensetzung über das Retentat zurückgehalten und wieder in die Reaktionszone zurückgeführt sowie Abbauprodukte der in der Hydroformylierung katalytisch aktiven Zusammensetzung über das Permeat entfernt werden.

Außerdem wird beschrieben:

2.) eine Vorrichtung zur Steuerung der Viskosität von Reaktionslösungen in der Hydroformylierung von olefinhaltigen Gemischen umfassend:

i) mindestens 1 Reaktionszone;
ii) Verdichter;
iii) Druckhaltung;
iv) mindestens 1 Kondensationszone, welche einen Wärmeaustauscher und nachgelagerten Sammelbehälter beinhaltet;
v) mindestens 1 Membranfiltration, dadurch gekennzeichnet, dass die Abbauprodukte der in der Hydroformylierung katalytisch aktiven Zusammensetzung über das Permeat aus dem Reaktionsgemisch abgetrennt, die in der Hydroformylierung katalytisch aktive Zusammensetzung über das Retentat zurückgehalten und wieder in die Reaktionszone geführt wird.

Eine Ausführungsform des erfindungsgemäßen Verfahrens umfasst als in der Hydroformylierung katalytisch aktive Zusammensetzung:

a) mindestens eine Organophosphorverbindung mit dreiwertigem Phosphor;
b) mindestens ein Metall aus den Gruppen 8 - 10 des Periodensystems der Elemente;
c) optional eine stabilisierende Komponente.

In einer besondern Ausführungsform des erfindungsgemäßen Verfahrens ist die Organophosphorverbindung mit dreiwertigem Phosphor ausgewählt unter Phosphinen, Phosphiten, Phosphoniten, Phosphiniten, Phosphoramiditen, das Metall ausgewählt ist aus der Gruppe 8 des Periodensystems der Elemente, die stabilisierende Komponente ausgewählt ist unter sterisch gehinderte Aminen.

In einer ganz besonderen Ausführungsform des erfindungsgemäßen Verfahrens ist das Metall Rhodium, das sterisch gehinderte Amin umfasst mindestens eine 2,2,6,6-Tetramethylpiperidin-Einheit.

In einer Ausführungsform des erfindungsgemäßen Verfahrens werden die Abbauprodukte der in der Hydroformylierung katalytisch aktiven Zusammensetzung, ausgewählt unter Alkoholen, Phenolen, Diolen, insbesondere Biphenolen, Binaphtholen, über das Permeat entfernt.

In einer weiteren Ausführungsform des erfindungsgemäßen Verfahrens wird die Membranfiltration:

1) in einem Temperaturbereich von 20 - 90°C;
2) bei einem transmembranen Druck im Bereich von 1,0 - 3,0 MPa;
3) bei einer Viskosität von maximal 10 mPas

durchgeführt.

In einer Ausführungsform des erfindungsgemäßen Verfahrens übersteigt das Molekulargewicht der über das Permeat abgetrennten Abbauprodukte der in der Hydroformylierung katalytisch aktiven Zusammensetzung nicht 400 g/mol, wobei es 20 - 50 % des Molgewichts der verwendeten Organophosphorverbindung beträgt

In einer Ausführungsform des erfindungsgemäßen Verfahrens ist der Rückhalt der Abbauprodukte der in der Hydroformylierung katalytisch aktiven Zusammensetzung im Retentat 80% oder kleiner, besonders bevorzugt 70% oder kleiner, und insbesondere 50% oder kleiner.

Nanofiltration ist ein druckgetriebenes Membrantrennverfahren. Die Trenngrenze (engl.: molecular weight cut-off, MWCO) liegt im Bereich von 150g/mol bis 2000g/mol. Über diesen Wert lässt sich die Nanofiltration von anderen Membrantrennverfahren wie Mikrofiltration und Ultrafiltration abgrenzen. Die Trenngrenze ist definiert als die Molmasse eines bevorzugt inerten Indikatorsystems (z. B. Polystyrolstandards oder Alkanstandards, vgl. Y.H. See Toh, X.X. Loh, A. Bismarck, A.G. Livingston, In search of a standard method for the characterisation of organic solvent nanofiltration membranes, J. Membr. Sci, 291(2007)120-125), bei der eine Membran einen Rückhalt von 90% aufweist.

Die genaue Trenngrenze einer Nanofiltrationsmembran ist von der eingesetzten Membran und dem jeweiligen Lösemittel sowie durch die Prozessbedingungen wie Druck und Temperatur bestimmt.
Somit unterscheiden sich die Trenngrenzen bei unterschiedlichen Lösungsmitteln teilweise stark. Im folgenden sind die genannten Trenngrenzen auf eine Bestimmung in Toluol mit Polystyrolstandards bei 30°C und 3,0 MPa transmembraner Druckdifferenz gemäß Y.H. See Toh, X.X. Loh, A. Bismarck, A.G. Livingston, [In search of a standard method for the characterisation of organic solvent nanofiltration membranes], J. Membr. Sci, 291(2007)120-125) zu verstehen.
In einer besondern Ausführungsform des erfindungsgemäßen Verfahrens weist die Membranfiltration bei einer Temperatur von 30°C und 3,0 MPa transmembranen Druck in Toluol im Bereich von 400 g/mol bis 500 g/mol zumindest punktuell einen Rückhalt von 90 % sowie im Bereich von 210 g/mol bis 310 g/mol zumindest punktuell einen Rückhalt von 60 % auf.
Bei der Nanofiltration werden dichte oder poröse Membranen eingesetzt. Nanofiltrationsmembranen zeichnen sich durch einen geringen Rückhalt für niedermolekulare organische Stoffe aus.

Der Rückhalt R einer Membran ist über die örtlichen Konzentrationen einer Komponente i des nicht permeierenden Stroms (Retentat) sowie des durch die Membran permeierenden Stroms (Permeat) bestimmt. Sind Retentat und Permeat entlang der Membran ideal durchmischt, so entsprechen die örtlichen Retentat- und Permeatkonzentrationen den jeweiligen Konzentration des in Summe anfallenden Retentats bzw. Permeats. Der Rückhalt R einer Membran für eine im zugeführten Stoffstrom enthaltene Komponente i ist im allgemeinen wie folgt definiert:

$$R = 1 - cPi/cRi$$

Dabei ist cPi die Konzentration der Komponente i im Permeat P und cRi die Konzentration der Komponente i im Retentat R. Im Grenzfall eines vollständigen Rückhalts der Komponente i durch die Membran ist cPi = 0 und R = 1. Im Fall einer bevorzugten Permeation der Komponente i ist cPi > cRi, und R < 0.

Vorzugsweise werden Membranen eingesetzt, die eine trennaktive Schicht aus Materialien ausgewählt aus Celluloseacetat, Cellulosetriacetat, Cellulosenitrat, regenerierter Cellulose, Polyimiden, Polyamiden, Polyetheretherketonen, sulfonierten Polyetheretherketonen, aromatischen Polyamiden, Polyamidimiden, Polybenzimidazolen, Polybenzimidazolone, Polyacrylnitril, Polyarylethersulfone, Polyester, Polycarbonaten, Polytetrafluorethylen, Polyvinylidenfluorid, Polypropylen, Polydimethylsiloxan, Silicone, Polyphosphazene, Polyphenylsulfide, Polybenzimidazole, 6.6 Nylon, Polysulfone, Polyaniline, Polypropylene, Polyurethane, Acrylonitril/Glycidylmethacrylat (PANGMA), Polytrimethylsilylpropyne, Polymethylpentyne, Polyvinyltrimethylsilan, Polyphenylenoxid, y-Aluminiumoxide, $\alpha$-Aluminiumoxide, Titaniumdoxide, Siliciumoxide, Zirconiumoxide, mit Silanen hydrophobisierte keramische Membranen, wie sie in DE 103 08 111 beschrieben sind, Polymere mit intrinsischer Mikroporosität (PIM) wie PIM-1 und anderen, wie sie z. B. in EP 0 781 166, WO 2010/097376 A1 und in "Membranes" von I. Cabasso, Encyclopedia of Polymer Sience and Technlogy, John Wiley and Sons, New York, 1987, beschrieben werden, aufweisen.

Die oben genannten Stoffe können insbesondere in der trennaktiven Schicht gegebenenfalls durch Zugabe von Hilfsstoffen vernetzt vorliegen oder als so genannte Mixed Matrix Membranen mit Füllstoffen wie z.B. Carbon Nano Tubes, Metal Organic Frameworks oder Hollow Spheres sowie Partikel von anorganischen Oxiden oder anorganische Fasern, wie z. B. Keramik- oder Glasfasern versehen sein.

Besonders bevorzugt werden Membranen eingesetzt, die als trennaktive Schicht eine Polymerschicht aus Polydimethylsiloxan, Polyimid, Polyamidimid, Acrylonitril/Glycidylmethacrylat (PANGMA), Siliconacrylat, endständig und/oder seitenständig organomodifiziertes Siloxan, Polyamid oder Polyetheretherketon aufweisen, die aus Polymeren mit intrinsischer Mikroporosität (PIM) wie PIM-1 aufgebaut sind, oder wobei die trennaktive Schicht über eine hydrophobierte keramische Membran aufgebaut ist. Ganz besonders bevorzugt werden Membranen aus Silikonen oder Polyamidimid eingesetzt. Solche Membranen sind kommerziell erhältlich.

In einer weiteren Ausführungsform der beschriebenen Vorrichtung ist das Material der Nanofiltrationsmembran ausgewählt aus:

a) Polydimethylsiloxan;
b) Siliconacrylat;
c) endständig und/oder seitenständig organomodifiziertes Siloxan;
d) Polyimid;
e) PIM-1.

Neben den oben genannten Materialien können die Membranen weitere Materialien aufweisen. Insbesondere können die Membranen Stütz- oder Trägermaterialien aufweisen, auf die die trennaktive Schicht aufgebracht ist. Bei solchen Verbundmembranen liegt neben der eigentlichen Membrane noch ein Stützmaterial vor. Eine Auswahl von Stützmaterialien beschreibt EP 0 781 166, auf welche explizit verwiesen wird.

Eine Auswahl von kommerziell erhältlicher Organic-Solvent-Nanofiltration Membranen sind die MPF und Selro Serien von Koch Membrane Systems, Inc., unterschiedliche Typen von Solsep BV, die Starmem™ Serie von Grace/UOP, die DuraMem™ und PuraMem™ Serie von Evonik Industries AG, die Nano-Pro Serie von Bio-Pure Technology, die HITK-T1 von IKTS, sowie oNF-1, oNF-2 und NC-1 von der GMT Membrantechnik GmbH.

Besonders bevorzugt umfasst die bei dem erfindungsgemäßen Verfahren und der beschriebenen Vorrichtung einzusetzende Nanofiltrationseinrichtung eine oder mehrere Nanofiltrationsmembranen, wobei mindestens eine der Nanofiltrationsmembranen eine Trenngrenze im Bereich von 150 bis 2000 g/mol, bevorzugt 200 bis 600 g/mol besonders bevorzugt 350 bis 500 aufweist.

Die Membranen werden in dem erfindungsgemäßen Verfahren vorzugsweise in Form von Membranmodulen eingesetzt. In diesen Modulen werden die Membranen so angeordnet, dass die Retentatseite der Membran derart überströmt werden kann, dass die Konzentrationspolarisation der abgetrennten Komponenten, hier Katalysator-Ligandsystem, entgegengewirkt und außerdem die nötige treibende Kraft bzw. Druck aufgeprägt werden kann. Das Permeat wird im Permeatsammelraum auf der Permeatseite der Membran zusammengeführt und aus dem Modul abgeführt. Gängige Membranmodule weisen die Membrane in Form von Membranscheiben, Membrankissen oder Membrantaschen auf. Im erfindungsgemäßen Verfahren werden die Membrane vorzugsweise in Form von Membranmodulen eingesetzt, die Membranmodule mit offenkanaligen Kissenmodulsystemen, bei denen die Membranen zu Membrantaschen oder -kissen thermisch verschweißt oder verklebt sind, oder offenkanaligen (Wide-spacer) Wickelmodulen, bei denen die Membranen zu Membrantaschen oder Membrankissen verklebt oder verschweißt sind und mit Feed-spacern um ein Permeatsammelrohr aufgewickelt sind, aufweisen.

Überströmung, Trennschritt

Um Ablagerungen auf der Membran zu vermeiden sind innerhalb der Membrantrennschritte gewisse Strömungsverhältnisse einzuhalten. Es hat sich gezeigt, dass die Ablagerungsgefährdung einer Strömung von deren Turbulenz und damit von ihrer Reynolds-Zahl abhängig ist. So ist unabhängig von der Bauart des Membranmoduls darauf zu achten, dass die Reynoldszahl zwischen 55 und 13500, bevorzugt zwischen 100 und 3500 und ganz besonders bevorzugt zwischen 170 und 900 liegt. Die Viskosität sollte dabei kleiner als 10 mPas und bevorzugt bei 1 mPas liegen. Bei diesen Strömungsverhältnissen werden Ablagerungen vermieden.

Die dimensionslose Reynolds-Zahl Re ist definiert als $Re = w \cdot dh/v$, wobei v die kinematische Viskosität, w die mittlere Überstromgeschwindigkeit der Membran und dh den hydraulischen Durchmesser als charakteristische Länge des Membranmoduls beschreibt. Die Bestimmung des hydraulischen Durchmessers für Spiralwickelelemente ist in G. Schock et al. "Mass transfer and pressure loss in spiral wound modules", Desalination, 64 (1987) 339-352 beschrieben. Zur Umsetzung dieser Strömungsverhältnisse wird bei der Verwendung von Wickelmembranen mit einer Rohrlänge von 1 m und einem Druckverlust von 0,15 MPa und einer kinematischen Viskosität des Mediums von 1 mPas zur Vermeidung von Ablagerungen auf der Membran das Verfahren vorzugsweise so durchgeführt, dass der Membrantrennschritt, insbesondere der erste Membrantrennschritt mit einer Überströmgeschwindigkeit an der Membran von 0,1 bis 15 m/sec., bevorzugt 0,2 bis 4 m/sec, vorzugsweise von 0,3 bis 1 m/sec vorliegt.

Vorzugsweise wird das erfindungsgemäße Verfahren derart betrieben, dass die zu trennende Lösung als Zustrom auf die Membran gefahren und der Retentatstrom teilweise auf die Membran zurückgeführt wird. Dabei wird der Teilstrom, der auf die Membran zurückgeführt wird, zuvor mit der zu trennenden Lösung vereinigt. Der Teil des Retentatstroms, der nicht auf die Membran zurückgeführt wird, wird entweder als Zufuhrstrom für eine oder mehrere nachfolgende Abtrennstufen verwendet oder aber in die Reaktion zurückgeführt.
Wird dem Membrantrennschritt ein Strom mit geringem Anteil an Hochsiedern und hohem Anteil an primären Produkten, wie dies bei einem Reaktoraustrag ohne vorherige Einengung von Hochsiedern der Fall ist, zugeführt, beträgt das Volumenstrom Verhältnis von Permeatstrom zu Zufuhrstrom vom Reaktor (ohne rückgeführtem Retentat) 1 zu 1,1 bis

1 zu 5, bevorzugt von 1 zu 1,4 bis 1 zu 3 und besonders bevorzugt von 1 zu 1,6 bis 1 zu 2.

Wird im umgekehrten Fall dem Membrantrennschritt ein nach dem Reaktor, z.B. durch einen thermischen Trennschritt, ein gegenüber dem Reaktoraustrag an Hochsieder deutlich angereicherter Strom zugeführt, so beträgt das Volumenstrom-Verhältnis von Permeatstrom zu Zufuhrstrom vom Reaktor (ohne rückgeführtem Retentat) vorzugsweise 1 zu 5 bis 1 zu 20, bevorzugt von 1 zu 7,5 bis 1 zu 12,5 und besonders bevorzugt von 1 zu 9 bis 1 zu 11.

Es kann vorteilhaft sein, wenn der Volumenstrom, der über die Membran geführt wird, deutlich größer ist als der Volumenstrom des Permeatstroms, da auf diese einfache Weise eine hohe Überströmgeschwindigkeit an der Membran eingestellt werden kann. Vorzugsweise beträgt das Volumenstromverhältnis des auf die Membran, insbesondere auf die erste Membran des ersten Membrantrennschritts geführten Stroms (Zufluss vom Reaktor inklusive rückgeführtem Retentat) zu Permeatstrom von 10 bis 10000 zu 1, bevorzugt von 50 bis 5000 zu 1 und besonders bevorzugt von 200 bis 2000 zu 1. Es wird also vorzugsweise ein relativ hoher Volumenstrom im Kreis über die Membran geführt. Die Größe des Teils des Retentatstroms, der in die Reaktion zurückgeführt oder einer weiteren Abtrennung zugeführt wird, ergibt sich aus der Differenz von Zufuhrstrom (ohne rückgeführtem Retentat) und Permeatstrom.

Bei höheren Permeabilitäten kann es auch vorteilhaft sein, die Membranen in einer Tannenbaumstruktur zu verschalten.

Als Zuführstrom zur Membrantrennung kann der Reaktionsaustrag einer durch organische Metallkomplexe katalysierte Reaktion direkt oder ein daraus hergestelltes Konzentrat eingesetzt werden. Die Reaktionsausträge enthalten Edukte, primäre Produkte, Nebenprodukte wie beispielsweise Ligandenabbauprodukte, die in der Hydroformylierung katalytisch aktive Zusammensetzung und gegebenenfalls ein Lösungsmittel. Wenn dieses Gemisch erfindungsgemäß aufgearbeitet wird, verbleibt die katalytisch aktive Zusammensetzung, insbesondere der Metallkomplex, vorwiegend im Retentat. Mit dem Permeat, das in einer weiteren Trennstufe aufgearbeitet wird, werden Edukte, Produkte und Ligandenabbauprodukte zusammen abgetrennt. In diesem Falle ist der Permeatstrom wesentlich größer als der Retentatstrom, der nicht auf die Membran zurückgeführt wird. Dies bedingt eine große Membranfläche und einen nicht optimalen Rückhalt der katalytisch aktiven Zusammensetzung.

**Beschreibung der und des erfindungsgemäßen Verfahrens**

Eine Ausführungsform der Vorrichtung wird in Figur 1 dargestellt. Die Anlage besteht aus drei Reaktoren (A1 bis A3). In den ersten Reaktor (A1) werden das flüssige Edukt(1), das Synthesegas(2), wahlweise frische Liganden- oder Katalysatorlösung (15) und der Katalysatorrückstrom aus der Nanofiltration (6) eingespeist. Die Reaktoren (A1 bis A3) sind über eine Gaspendelleitung (10) miteinander verbunden. Die flüssige Phase wird über einen Überlauf im Kopfbereich jeden Reaktors zum Fuß des nächsten Reaktors geleitet (12). Am Überlauf des letzten Reaktors (A3) wird der flüssige Produktstrom (8) in den Strippbehälter(B1) gefahren. Über einen Doppelmantel und einer innen liegenden Rohrschlange kann der Strippbehälter (B1) beheizt werden. Die Temperatur im Strippbehälter (B1) kann zwischen 80°C und 140°C gefahren werden. Der Druck im Strippbehälter (B1) kann zwischen 5 bar und 17bar gefahren werden. Der Abgasstrom der Reaktoren (A1 bis A3) wird druckgeregelt in den Strippbehälter (B1) gefahren.

Hierbei kann der Abgasstrom (14) wahlweise mit dem Produktstrom(8) zuvor vereint werden, separat in den Strippbehälter (B1) gefahren (gestrichelte Linie) werden oder aber dem Sumpf des Strippbehälters (B1) zugeführt (gestrichelte Linie) werden. Die Reaktoren (A1 bis A3) verfügen über getrennte Möglichkeiten diese zu heizen oder zu kühlen.

Hierzu sind die Reaktoren mit einem Doppelmantel und zusätzlichen innenliegenden Rohrschlangen ausgestattet.

Aus dem Strippbehälter (B1) wird die Gasphase (7) über einen Kondensator (B2) gefahren. Hierbei wird ein Großteil der kondensierbaren Komponenten aus dem Gasstrom auskondensiert und im Phasentrennbehälter (B3) abgeschieden. Die flüssige Phase(5) aus dem Phasentrennbehälter (B3) wird der weiteren Aufarbeitung zugeführt.

Die Gasphase (11) aus dem Phasentrennbehälter (B3) wird einem Verdichter (B4) zugeführt, der den Gasstrom(9) wieder in den Sumpf des Strippbehälters (B1) zurückführt, um so die Trennung von Produkt und Katalysatorstrom unter Druck zu ermöglichen. Das überschüssige Gas (3) wird druckgeregelt aus dem System gefahren und einer weiteren Verwendung zugeführt.

Der Sumpfstrom (13) des Strippbehälters (B1) wird über eine Pumpe wieder auf den Reaktionsdruck in den Reaktoren (A1 bis A3) gebracht und der Nanofiltration (C1) - dargestellt in Figur 3 - zugeführt. Hier wird der Strom in den Katalysatorrückstrom (6) und dem Ausschleusestrom (4) aufgeteilt. Der Ausschleusestrom (4) wird einer weiteren Aufarbeitung zugeführt.

**Verfahrens- und Vorrichtungsalternative**

Eine weitere Ausführungsform der Vorrichtung wird in Figur 4 dargestellt. Die Anlage besteht aus drei Reaktoren(A1 bis A3). In jeden der Reaktoren (A1 bis A3) werden das Edukt(1), in flüssiger oder gasförmiger Form, das Synthesegas(2), wahlweise frische Liganden- oder Katalysatorlösung (15) und der Katalysatorrückstrom aus der Nanofiltration(6) eingespeist. Die Produktentfernung aus den Reaktoren (A1 bis A3) erfolgt über einen Kreisgasstrom, der das Produkt aus der Reaktionsmischung über den Partialdruckanteil entfernt. Hierzu wird ein Kreisgasstrom(9) über den Verdichter(B4) in den Sumpf jeden Reaktors(A1 bis A3) gefahren. Das produktgesättigte Gas(7) verlässt die Reaktoren am Kopfund wird einem Kondensator(B2) zugeführt. Hier wird das produktgesättigte Gas abgekühlt, so dass das Produkt teilweise auskondensiert. Das Produkt wird in Behälter(B3) gesammelt und der weiteren Aufarbeitung zugeführt.

Überschüssiges Gas wird über eine Druckhaltung(3) aus dem System entfernt.

Aus jedem Reaktor wird ein Teilstrom der Reaktionslösung entnommen(13) und der Nanofiltrationsanlage(C1) - dargestellt in Figur 3 - zugeführt. Der resultierende Permeatstrom(4) wird einer weiteren Aufarbeitung zugeführt. Der Retentatstrom(6) wird wieder den Reaktoren(A1-A3) zugeführt.

In einer Ausführungsform der Vorrichtung ist die Kondensationszone, welche einen Wärmeaustauscher oder Kondensator B(2) und einem Sammelbehälter B(3) beinhaltet, in einer Baugruppe zusammengefasst.

**Beispiele**

**Abkürzungen:**

acac = Acetylacetonat

**Versuchsbeschreibung - allgemein**

Die Versuche wurden in 500 ml-Autoklaven der Fa. Parr Instrument durchgeführt. Die Autoklaven sind mit einer elektrischen Beheizung ausgerüstet. Die Druckkonstanthaltung erfolgt über Massedurchflußmesser und Druckregler Über Kapillarleitungen und HPLC-Ventile können während der Versuchszeit Proben gezogen und sowohl über GC- als auch über LC-MS-Analytik untersucht werden.

Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Um die Zusammenhänge zwischen Viskosität der Reaktionslösung und den darin vorliegenden Verbindungen wie auch deren Gehalten zu demonstrieren, wurden die folgenden Beispiele I bis XI ausgeführt.

Aus dem Stand der Technik, wie z. B. in EP 1 430 014 B1 dargelegt, ist bekannt, dass die Viskosität von Zusammensetzungen sich in guter Näherung aus den Einzelkomponenten der zu untersuchenden Lösung gemäß ihres Anteils an dieser Lösung zusammensetzt. Dies gilt bei Systemen, die ein newtonsches Verhalten aufweisen.

Beispiel XI ist an den in WO 2010/003073 A1 offenbarten Stand der Technik angelehnt und dient als Vergleichsbeispiel.

**Beispiel I**

In einem, mit Argon inertisierten, 500ml Autoklaven wurden 200ml Aldehyde, mit einer Zusammensetzung von 5% 2-Methylbutanal und 95% Pentanal, unter 1,7 MPa $CO/H_2$ (50Vol%/50Vol%) Atmosphäre einer Temperatur von 120°C ausgesetzt. Aus dem Autoklaven wurde je 4ml Probe entnommen und die Viskosität vermessen. Die entnommene Probemenge wurde durch Aldehyd ersetzt. Der Aldehyd hat die gleiche Zusammensetzung wie das am Anfang eingesetzte Aldehyd. Nach 1800h wurde der Versuch beendet und der Aldehydumsatz per GC bestimmt. Der Umsatz der C5 Aldehyde belief sich auf 95%. Die Analyse zeigte, dass die Aldehyde zu Hochsiedern umgesetzt wurden. Bei dem Hauptprodukt handelte es sich um das kondensierte Produkt der entsprechenden C10 Aldole. Bei der Entleerung des Autoklavens wurde eine Wasserphase gefunden.

(Die Abnahme der Viskosität ist durch die Kondensation der Aldolprodukte zu erklären. Nach 650h wird eine Viskosität von 14mPas gemessen, dies entspricht der Viskosität der nicht kondensierten Aldolprodukte. Durch die anschließende Wasserabspaltung wird das sogenannte C10-enal gebildet, dadurch kommt es zu einer Viskositätsabsenkung in diesem Versuch.)

Tab001

| Zeit in h | Viskosität in mPas |
|---|---|
| 0 | 0,5 |

(fortgesetzt)

| Zeit in h | Viskosität in mPas |
|---|---|
| 650 | 14 |
| 1000 | 8 |
| 1200 | 5 |
| 1400 | 2 |
| 1500 | 2 |
| 1700 | 2 |

**Beispiel II**

Im einem 500ml Rundkolben mit Rückflußkühler und Wasserauskreiser, wurden unter Argonatmosphäre 200ml Aldehyde, mit einer Zusammensetzung von 95% Pentanal und 5% 2-Methylbutanal unter Normaldruck im Rückfluss gekocht. Entstehendes Wasser wurde am Wasserauskreiser aus dem System entfernt.

Aus der Apparatur wurde je 4ml Probe entnommen und die Viskosität vermessen. Die entnommene Probemenge wurde durch Aldehyd ersetzt. Der Aldehyd hat die gleiche Zusammensetzung wie das am Anfang eingesetzte Aldehyd. Nach 1150h wurde der Versuch beendet und der Aldehydumsatz per GC bestimmt.

Der Umsatz der C5-Aldehyde belief sich auf 95%.

Die Analyse zeigte, dass die Aldehyde zu Hochsiedern umgesetzt wurden. Bei dem Hauptprodukt handelte es um das Tetramere Produkt aus dem C5 Aldehyden. Das in Beispiel I gezeigte kondensierte Produkt wurde nur im unteren Prozentbereich detektiert.

Tab002

| Zeit in h | Viskosität in mPas |
|---|---|
| 0 | 0,5 |
| 180 | 7 |
| 600 | 7 |
| 800 | 6 |
| 1000 | 7 |
| 1150 | 6 |

Da die entsprechenden Hochsieder der Aldhehyde offensichtlich nicht für einen Viskositätsanstieg verantwortlich sind, wurden, neben den aus dem Liganden 5 b, auch bekannt unter dem Kurznamen Biphephos oder unter (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine)), entstehenden Phenolen ([1,1'-biphenyl]-2,2'-diol und 3,3'-di-tert-butyl-5,5'-dimethoxy-[1,1'-biphenyl]-2,2'-diol), verschiedene Derivate auf ihren Einfluss auf die Viskosität der Reaktionsmischung vermessen.

Diese Biphenolderivate können in bekannter Weise für die Synthese von Phosphitliganden verwendet werden.

Ebenso wurde der in Beispiel XI, XII und XIII eingesetzte Stabilisator vermessen, um dessen Einfluss auf die Viskosität festzustellen.

**Beispiel III**

In Texanol (2,2,4-Trimethyl-1,3-pentandiol-monoisobutyrat), dem Trimeren Additionsprodukt aus den C4 Aldehyden gemäß CAS Reg. Nr. 25265-77-4, wurden jeweils Lösungen von 2,2' Dihydroxybiphenyl, Komponente A der Ligandenabbauprodukte, angesetzt und die Viskosität der Lösung bei Raumtemperatur vermessen.

Tab003

| 2,2' Dihydroxybiphenyl w% in Texanol | Viskosität in mPas |
|---|---|
| 0 | 17 |

(fortgesetzt)

| 2,2' Dihydroxybiphenyl w% in Texanol | Viskosität in mPas |
|---|---|
| 2 | 20 |
| 3 | 22 |
| 6 | 25 |
| 10 | 32 |
| 20 | 78 |

**Beispiel IV**

In Texanol, dem Trimeren Additionsprodukt aus den C4 Aldehyden gemäß CAS Reg. Nr. 25265-77-4, wurden jeweils Lösungen von Bis (2,2,6,6-Tetramethyl-4-Piperidinyl) sebacate angesetzt und die Viskosität der Lösung bei Raumtemperatur vermessen.

Tab004

| Bis (2,2,6,6-Tetramethyl-4-Piperidinyl) sebacate w% in Texanol | Viskosität in mPas |
|---|---|
| 0 | 17 |
| 2 | 19 |
| 5 | 21 |
| 10 | 30 |
| 20 | 44 |

**Beispiel V**

In Texanol, dem Trimeren Additionsprodukt aus den C4 Aldehyden gemäß CAS Reg. Nr. 25265-77-4, wurden die Löslichkeitsgrenze von 3,3'-di-tert-butyl-5,5'-dimethoxy-[1,1'-biphenyl]-2,2'-diol, Komponente B der Ligandenabbauprodukte, mit Hilfe einer HPLC-Analyse ermittelt. Dazu wurden 5g 3,3'-di-tert-butyl-5,5'-dimethoxy-[1,1'-biphenyl]-2,2'-diol bei Raumtemperatur unter rühren in 50g Texanol gegeben. Die Lösung wurde über Nacht gerührt. Am nächsten Morgen war die Lösung immer noch trüb. Nachdem der Rührer abgestellt wurde, bildete sich ein Bodenkörper. Aus der überstehenden klaren Lösung wurde eine Probe entnommen und mit HPLC analysiert. Die Analyse ergab, das sich 0,4g/l 3,3'-di-tert-butyl-5,5'-dimethoxy-[1,1'-biphenyl]-2,2'-diol in der Lösung befanden. Der Bodenkörper wurde aus dem Ansatz abfiltriert und in THF aufgelöst. Eine Probe dieser Lösung wurde mit einem GC-MS analysiert und ergab, dass es sich bei dem Feststoff um 3,3'-di-tert-butyl-5,5'-dimethoxy-[1,1'-biphenyl]-2,2'-diol handelte.

**Beispiel VI**

In Texanol gemäß CAS Reg. Nr. 25265-77-4, dem Trimeren Additionsprodukt aus den C4 Aldehyden, wurden jeweils Lösungen von 3,3',5,5'-tetramethyl-[1,1'-biphenyl]-2,2'-diol, angesetzt und die Viskosität der Lösung bei Raumtemperatur vermessen.

Tab005

| 3,3',5,5'-tetramethyl-[1,1'-biphenyl]-2,2'-diol w% in Texanol | Viskosität in mPas |
|---|---|
| 0 | 14 |
| 2 | 15 |
| 5 | 17 |
| 10 | 21 |
| 20 | 35 |

**Beispiel VII**

In Texanol, dem Trimeren Additionsprodukt aus den C4 Aldehyden gemäß CAS Reg. Nr. 25265-77-4, wurden jeweils Lösungen von 2,4-dimethylphenol, angesetzt und die Viskosität der Lösung bei Raumtemperatur vermessen.

Tab006

| 2,4-dimethylphenol w% in Texanol | Viskosität in mPas |
|---|---|
| 0 | 14 |
| 2 | 14,1 |
| 5 | 14,3 |
| 10 | 15,3 |
| 20 | 17,2 |

**Bespiel VIII**

In Texanol, dem Trimeren Additionsprodukt aus den C4 Aldehyden gemäß CAS Reg. Nr. 25265-77-4, wurden die Löslichkeitsgrenze von 3,3',5,5'-tetrakis-tert-butyl-[1,1'-biphenyl]-2,2'-diol mit Hilfe einer HPLC-Analyse ermittelt.

Dazu wurden 5g 3,3',5,5'-tetrakis-tert-butyl-[1,1'-biphenyl]-2,2'-diol bei Raumtemperatur unter rühren in 50g Texanol gegeben. Die Lösung wurde über Nacht gerührt. Am nächsten Morgen war die Lösung immer noch trüb. Nachdem der Rührer abgestellt wurde, bildete sich ein Bodenkörper. Aus der überstehenden klaren Lösung wurde eine Probe entnommen und mit HPLC analysiert. Die Analyse ergab, das sich 0.4g/l 3,3',5,5'-tetrakis-tert-butyl-[1,1'-biphenyl]-2,2'-diol in der Lösung befanden. Der Bodenkörper wurde aus dem Ansatz abfiltriert und in THF aufgelöst. Eine Probe dieser Lösung wurde mit einem GC-MS analysiert und ergab, dass es sich bei dem Feststoff um 3,3',5,5'-tetrakis-tert-butyl-[1,1'-biphenyl]-2,2'-diol handelte.

**Bespiel IX**

In Texanol, dem Trimeren Additionsprodukt aus den C4 Aldehyden gemäß CAS Reg. Nr. 25265-77-4, wurden jeweils Lösungen von [1,1'-binaphthalene]-2,2'-diol, angesetzt und die Viskosität der Lösung bei Raumtemperatur vermessen.

Tab007

| [1,1'-binaphthalene]-2,2'-diol w% in Texanol | Viskosität in mPas |
|---|---|
| 0 | 14 |
| 2 | 15 |
| 5 | 16 |
| 10 | 19,5 |
| 20 | 29 |

**Bespiel X**

In Texanol, dem Trimeren Additionsprodukt aus den C4 Aldehyden gemäß CAS Reg. Nr. 25265-77-4, wurden jeweils Lösungen von 2- Methyl-4,6- di.tert-butylphenol, angesetzt und die Viskosität der Lösung bei Raumtemperatur vermessen.

Tab008

| 2- Methyl-4,6- di.tert-butylphenol w% in Texanol | Viskosität in mPas |
|---|---|
| 0 | 14 |
| 2 | 14,2 |

(fortgesetzt)

| 2- Methyl-4,6- di.tert-butylphenol w% in Texanol | Viskosität in mPas |
|---|---|
| 3 | 15,0 |
| 6 | 16,4 |
| 10 | 19,0 |
| 20 | 26,2 |

Zusammenfassung

Die Beispiele I und II zeigen, das es im Laufe der Reaktion von den eingesetzten Aldehyden zu keinem schädlichen Anstieg der Viskosität durch deren Reaktionsprodukte kommt, wie es deutlich in den Tabellen Tab001 und Tab002 abzulesen ist. Somit haben diese Reaktionsprodukte keinen negativen Einfluss auf die Viskosität.

In den Beispielen III, IV, VI, VII, IX, X werden in den Tabellen Tab003, Tab004, Tab005, Tab006, Tab007 und Tab008 deutlich der Einfluss der einzelnen Komponenten auf die Viskosität der Lösung gezeigt. Mit steigender Konzentration der Komponenten ändert sich auch die resultierende Viskosität der Lösung.

In den Beispielen V und VIII zeigten die Komponenten eine zu geringe Löslichkeit im verwendeten Lösemittel. Obwohl hier keine Viskositätsmessungen durchgeführt werden konnten, ist dieser Feststoffanfall für die Reaktionsführung in der Hydroformylierung nicht erwünscht.

**Beispiel XI - Vergleichsbeispiel (nicht-erfinderisch)**

Mit der in Figur 2 abgebildeten Anlage - in Anlehnung an den Stand der Technik der WO 2010/003073 A1 - wurde eine kontinuierliche Hydroformylierungsreaktion durchgeführt. Hierbei wurden die Reaktoren je mit 7232g einer Katalysatorlösung gefüllt. Diese Katalysatorlösung bestand aus 7083g Isononylbenzoat, 42g Ligand 5 b, 103g Bis (2,2,6,6-Tetramethyl-4-Piperidinyl) sebacate , 4g $Rh(acac)(CO)_2$.

Die Reaktoren (A1 bis A3) wurden mit Synthesegas(2), mit einer Zusammensetzung von 50Vol% CO und 50Vol% $H_2$, auf 1,7 MPa(abs) aufgedrückt und auf 100°C aufgeheizt. Dann wurde der Eduktzulauf(1) gestartet. Pro Stunde wurden 1,9kg einer Mischung von 25% bis 35% 2-Butenen in 75% bis 65% n-Butan in den ersten Reaktor(A1) dosiert.

Zeitgleich wurde Synthesegas(2) in der schon beschriebenen Zusammensetzung auf den ersten Reaktor(A1) gegeben, um den Reaktionsdruck von 1,7 MPa(abs) zu halten.

Der Strippbehälter(B1) wurde auf 120°C vorgeheizt und die Kondensationstemperatur im Kondensator (B2) auf 40°C eingestellt.

Das Strippsystem, bestehend aus dem Strippbehälter (B1), Kondensator(B2), Phasentrennbehälter (B3) und Verdichter (B4) mit allen verbundenen Leitungen(14, 7, 9, 11, 13) wurden über die Abgasleitung (14) der Reaktoren bis auf 1,0 MPa(abs) aufgedrückt. Hierzu wurde das Synthesegas (2) am ersten Reaktor erhöht, um den Reaktionsdruck bei 1,7 MPa(abs) zu halten.

Bei erreichen des Überlaufes im dritten Reaktor (A3) wurde über die Standregelung(8) die flüssige Phase in den Strippbehälter (B1) geleitet.

Gleichzeitig wurde der Synthesegasstrom(2) auf den 1,1 fachen molaren Überschuss zum Butenstrom(1) angepasst. Über die Druckhaltung der Reaktoren(14) wurde der überschüssige Gasanteil in den Strippbehälter (B1) gefahren und dort über die Druckhaltung im Strippsystem (3), aus der Anlage gefahren. Der Druck in den Reaktoren(A bis A3) wurde so auf 1,7 MPa(abs) und im Strippsystem(B1 bis B4) auf 1,0 MPa(abs) gehalten.

Bei erreichen von 20% Stand in dem Strippbehälter(B1) wurde der Verdichter (B4) angefahren und das Produkt aus der flüssigen Phase mit Hilfe des erzeugten Gasstromes (9) ausgetrieben. Der Gasstrom (9) wurde entsprechend dem Stand im Strippbehälter (B1) angepasst. Bei steigendem Stand im Strippbehälter (B1) wurde der Gasstrom (9) erhöht und bei fallendem Stand im Strippbehälter (B1) wurde der Gasstrom (9) vermindert. Die notwendige Gasmenge variierte je nach Stand zwischen 300 und 1200l/h.

Aus dem Sumpf des Strippbehälters (B1) wurde ein Katalysatorstrom (13) von 900g/h über eine Pumpe zum ersten Reaktor (A1) zurückgefahren (6).

Am Reaktor (A3) wurde der Umsatz mittels GC bestimmt.

Am Strippbehälter (B1) wurde mit Hilfe eines HPLC der Anteil des ungebundenen Liganden bestimmt und dieser durch das Nachfahren einer 2%igen Ligandenlösung in entgastem Rohprodukt (15) auf einem konstanten Niveau gehalten. Das Verhältnis des ungebundenen Liganden 5 b, kurz Biphephos oder (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine) zum Rhodium wurde auf einem molaren

Verhältnis von 1:1 gehalten. Diese Lösung enthielt neben dem Liganden 5 b noch Bis (2,2,6,6-Tetramethyl-4-Piperidinyl) sebacate im molaren Verhältnis 2:1 Bis (2,2,6,6-Tetramethyl-4-Piperidinyl) sebacate zum Liganden 5 b, kurz Biphephos oder (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine).

Neben dem Ligandengehalt wurde auch die Viskosität der Reaktorlösung im Strippbehälter (B1) gemessen.

Nach ca.1500 h entsprechend 62,5 d Laufzeit stieg der Stand im Strippbehälter (B1) an und konnte auch durch ein Anheben der Gasmenge nicht mehr abgesenkt werden.

Zeitgleich war ein Anstieg der Viskosität zu beobachten. Der Versuch wurde noch bis 7500 h entsprechend 312,5 d Laufzeit weiter gefahren. Die Viskosität betrug am Ende der Laufzeit über 350 mPas und wies somit einen Anstieg um den Faktor 70 gegenüber den Startbedingungen auf; s. Figur 5. Es wurde ein Rückgang des Umsatzes beobachtet.

Beim Ablassen der Reaktionslösung aus dem Reaktionssystem wurde ein weißer Feststoff gefunden.

**Beispiel XII - gemäß der Erfindung**

Mit der in Figur 1 abgebildeten Anlage wurde eine kontinuierliche Hydroformylierungsreaktion durchgeführt.

Die Katalysatorlösung wurde analog zum Beispiel XI angesetzt

Die Reaktoren (A1 bis A3) wurden mit Synthesegas(2), mit einer Zusammensetzung von 50Vol% CO und 50Vol% $H_2$, auf 1,7 MPa(abs) aufgedrückt und auf 100°C aufgeheizt. Dann wurde der Eduktzulauf(1) gestartet. Pro Stunde wurden 1,9kg einer Mischung von 25% bis 35% 2-Butenen in 75% bis 65% n-Butan in den ersten Reaktor(A1) dosiert.

Zeitgleich wurde Synthesegas(2) in der schon beschriebenen Zusammensetzung auf den ersten Reaktor(A1) gegeben, um den Reaktionsdruck von 1,7 MPa(abs) zu halten.

Der Strippbehälter(B1) wurde auf 120°C vorgeheizt und die Kondensationstemperatur im Kondensator(B2) auf 40°C eingestellt.

Das Strippsystem, bestehend aus dem Strippbehälter(B1), Kondensator(B2), Phasentrennbehälter(B3) und Verdichter(B4) mit allen verbundenen Leitungen(14, 7, 9, 11, 13) wurden über die Abgasleitung(14) der Reaktoren bis auf 1,0 MPa(abs) aufgedrückt. Hierzu wurde das Synthesegas(2) am ersten Reaktor erhöht, um den Reaktionsdruck bei 1,7 MPa(abs) zu halten.

Bei erreichen des Überlaufes im dritten Reaktor(A3) wurde über die Standregelung(8) die flüssige Phase in den Strippbehälter(B1) geleitet.

Gleichzeitig wurde der Synthesegasstrom(2) auf den 1,1 fachen molaren Überschuss zum Butenstrom(1) angepasst. Über die Druckhaltung der Reaktoren(14) wurde der überschüssige Gasanteil in den Strippbehälter(B1) gefahren und dort über die Druckhaltung im Strippsystem(3), aus der Anlage gefahren. Der Druck in den Reaktoren(A1 bis A3) wurde so auf 1,7 MPa(abs) und im Strippsystem(B1 bis B4) auf 1,0 MPa(abs) gehalten.

Bei erreichen von 20% Stand in dem Strippbehälter(B1) wurde der Verdichter (B4)angefahren und das Produkt aus der flüssigen Phase mit Hilfe des erzeugten

Gasstromes(9) ausgetrieben. Der Gasstrom(9) wurde entsprechend dem Stand im Strippbehälter (B1) angepasst. Bei steigendem Stand im Strippbehälter (B1) wurde der Gasstrom(9) erhöht und bei fallendem Stand im Strippbehälter(B1) wurde der Gasstrom(9) vermindert. Die notwendige Gasmenge variierte je nach Stand zwischen 300 und 1200l/h. Aus dem Sumpf des Strippbehälters(B1) wurde ein Strom(13) von 900g/h über eine Pumpe zur zweistufigen Nanofiltrationseinheit(Figur 3) über den Zugang (A) in den Vorlagebehälter (M1) gefahren. Dort wurde die Lösung mit Synthesegas(B) überschichtet und der Druck oberhalb der Flüssigkeit auf 1,0 MPa(abs) gehalten. Überschüssiges Gas wurde druckgeregelt ins Abgas(C) gefahren. Das eingesetzte Synthesegas hat die gleiche Zusammensetzung wie das zur Reaktion eingesetzte Synthesegas. Auf den Vorlagebehälter(M1) wurde eine Synthesegasmenge von 45nl/h gegeben.

Aus der Vorlage (M1) wurde die flüssige Phase standgeregelt in die erste Membranstufe gefahren. Die Membranstufe bestand aus der Druckerhöhungspumpe (P1), der Überstrompumpe (P2), dem Wärmetauscher (W1) und dem Flachkanalmodul (M2). Die Überströmung über die Membran wurde mit der Pumpe (P2) auf 300l/h eingestellt. Die Temperatur in der ersten Membranstufe wurde mit dem Wärmeaustauscher(W1) so eingestellt, dass sich Rückhalte der Ligandenabbauprodukte, Komponente A und Komponente B, zwischen 10% und 70% über der Membran(M2) einstellten. Hierbei bewegten sich die Transmembrandrücke bei 1,0 bis 3,0 MPa.

Die Rückhalte der Abbaukomponenten, Komponente A und Komponente B, wurde durch Probennahme und HPLC-Analytik des Retentatstroms (E) und des Permeatstroms (G) ermittelt. Der Temperaturbereich in der Membranstufe wurde dazu zwischen 20°C und 90°C eingestellt.

Der Retentatstrom (E) der ersten Membranstufe wurde wieder zum ersten Reaktor (A1) zurückgefahren (6). Hierbei stelle sich ein Strom von 800-850g/h ein. Der Permeatstrom (G) aus der ersten Membranstufe wurde in den Vorlagebehälter (M3) der zweiten Membranstufe gefahren. Dieser Vorlagebehälter (M3) wurde ebenfalls analog zum ersten Vorlagebehälter (M1) mit Synthesegasdruck abgedeckt. Auf den Vorlagebehälter (M3) wurde eine Synthesegasmenge von 40nl/h gegeben.

Die flüssige Phase aus der Vorlage (M3) wurde ebenfalls standgeregelt in die zweite Membranstufe gefahren. Die zweite Membranstufe bestand aus der Druckerhöhungspumpe (P3), der Überströmpumpe (P4), dem Wärmeaustauscher (W2) und dem Flachkanalmodul (M4).

Die Überströmung über die Membran wurde mit der Pumpe (P4) auf 300l/h eingestellt.

Die Rückhalte der Abbaukomponenten, Komponente A und Komponente B, wurde durch Probennahme und HPLC-Analytik des Retentatstroms (E) und des Permeatstroms (G) ermittelt. Der Temperaturbereich in der Membranstufe wurde dazu zwischen 20°C und 90°C eingestellt.

Der Retentatstrom (F) der zweiten Membranstufe wurde wieder zum Vorlagebehälter (M1) der ersten Membranstufe zurückgefahren. Das Permeat (H) der zweiten Stufe wurde in die, ebenfalls synthesegasabgedeckte Vorlage (M5) gefahren. Aus dieser Vorlage (M5) wurde die flüssige Phase (D) aus dem System ausgeschleust. Dieser Strom betrug zwischen 50 und 100g/h.

Als Membranen wurde der PDMS- / Polydimethylsiloxan-basierte Typ oNF2 von GMT eingesetzt.

Die Nanofiltration wurde so betrieben, dass sich die Abbauprodukte des Liganden 5 b, kurz Biphephos oder (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine), Komponente A und Komponente B, nicht im System akkumulieren konnten; s. Figur 6.

Der Rückhalt berechnet sich wie folgt:

Rückhalt in % = (1- Konzentration der Komponente im Permeat)/ Konzentration der Komponente im Retentat.

Die Konzentrationen werden jeweils in g/l angegeben

Am Reaktor (A3) wurde der Umsatz mittels GC bestimmt.

Am Strippbehälter (B1) wurde mit Hilfe eines HPLC der Anteil des Liganden bestimmt und dieser durch das Nachfahren einer 2%igen Ligandenlösung in entgastem Rohprodukt (15) auf einem konstanten Niveau gehalten. Diese Lösung enthielt neben dem Liganden 5 b kurz Biphephos oder (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine) noch Bis (2,2,6,6-Tetramethyl-4-Piperidinyl) sebacate im molaren Verhältnis 2:1 Bis (2,2,6,6-Tetramethyl-4-Piperidinyl) sebacate zum Liganden Biphephos (6,6'-((3,3'-di-tert-butyl-5,5'-dimethoxy-[1,1'-biphenyl]-2,2'-diyl)bis(oxy))didibenzo[d,f][1,3,2]dioxaphosphepine).

Neben dem Ligandengehalt wurde auch die Viskosität der Reaktorlösung im Strippbehälter (B1) gemessen.

Auch nach über 1800h entsprechend 75 d Laufzeit des Versuches stieg der Stand im Strippbehälter(B1) nicht an. Es war auch kein Anstieg der Viskosität zu beobachten - s. Figur 7 -, welche im Versuchszeitraum unter 10 mPas blieb, und der in Beispiel XI aufgetretene Umsatzverlust wurde nicht beobachtet.

Nach 3500h entsprechend 146 d Laufzeit des Versuches wurde das Reaktionssystem ebenfalls entleert und es konnte kein Feststoff nachgewiesen werden.

**Beispiel XIII - gemäß der Erfindung**

Mit der in Figur 4 abgebildeten Anlage wurde eine kontinuierliche Hydroformylierungsreaktion durchgeführt.

Hierbei wurden die Reaktoren (A1-A3) je mit 3690g einer Katalysatorlösung gefüllt. Diese Katalysatorlösung bestand aus 3541g Isononylbenzoat, 42g Ligand 5 b, 103g Bis (2,2,6,6-Tetramethyl-4-Piperidinyl) sebacate , 4g $Rh(acac)(CO)_2$.

Die Reaktoren (A1 bis A3) wurden mit Synthesegas(2), mit einer Zusammensetzung von 50Vol% CO und 50Vol% $H_2$, auf 1,7 MPa(abs) aufgedrückt und auf 100°C aufgeheizt. Dann wurde der Eduktzulauf(1) gestartet. Pro Stunde wurden pro Reaktor 0,5kg einer Mischung von 25% bis 35% 2-Butenen in 75% bis 65% n-Butan flüssig in jeden der Reaktoren(A1-A3) dosiert. Zeitgleich wurde der Verdichter(B4) in Betrieb genommen und die Kondensationstemperatur im Kondensator(B2) auf 40°C eingestellt. Gleichzeitig wurde der Synthesegasstrom (2) auf den 1,1 fachen molaren Überschuss zum Butenstrom (1) angepasst. Über die Druckhaltung der Reaktoren (3) wurde der überschüssige Gasanteil aus der Anlage gefahren. Der Druck in den Reaktoren (A1 bis A3) wurde so auf 1,7 MPa(abs) gehalten.

Der Gasstrom (9) wurde entsprechend dem Stand in den Reaktoren (A1 bis A3) angepasst. Bei steigendem Stand in den Reaktoren (A1 bis A3) wurde der Gasstrom (9) erhöht und bei fallendem Stand in den Reaktoren (A1 bis A3), wurde der Gasstrom (9) vermindert. Die notwendige Gasmenge variierte je nach Stand zwischen 100 und 400l/h pro Reaktor.

Aus dem Sumpf der Reaktoren (A1 bis A3) wurde ein Strom (13) von 300g/h je Reaktor(A1-A3) über eine Pumpe zur zweistufigen Nanofiltrationseinheit (Figur 3) über den Zugang (A) in den Vorlagebehälter (M1) gefahren. Dort wurde die Lösung mit Synthesegas(B) überschichtet und der Druck oberhalb der Flüssigkeit auf 1,0 MPa(abs) gehalten. Überschüssiges Gas wurde druckgeregelt ins Abgas (C) gefahren. Das eingesetzte Synthesegas hat die

gleiche Zusammensetzung wie das zur Reaktion eingesetzte Synthesegas. Auf den Vorlagebehälter (M1) wurde eine Synthesegasmenge von 45nl/h gegeben.

Aus der Vorlage (M1) wurde die flüssige Phase standgeregelt in die erste Membranstufe gefahren. Die Membranstufe bestand aus der Druckerhöhungspumpe (P1), der Überstrompumpe(P2), dem Wärmetauscher (W1) und dem Flachkanalmodul (M2). Die Überströmung über die Membran wurde mit der Pumpe (P2) auf 300l/h eingestellt. Die Temperatur in der ersten Membranstufe wurde mit dem Wärmeaustauscher (W1) so eingestellt, dass sich Rückhalte der Ligandenabbauprodukte, Komponente A und Komponente B, zwischen 10% und 70% über der Membran (M2) einstellten. Hierbei bewegten sich die Transmembrandrücke bei 1,0 bis 3,0 MPa.

Die Rückhalte der Abbaukomponenten, Komponente A und Komponente B, wurde durch Probennahme und HPLC-Analytik des Retentatstroms (E) und des Permeatstroms (G) ermittelt. Der Temperaturbereich in der Membranstufe wurde dazu zwischen 20°C und 90°C eingestellt.

Der Retentatstrom (E) der ersten Membranstufe wurde wieder zu gleichen Teilen in die Reaktoren (A1-bis A3) zurückgefahren (6). Hierbei stelle sich ein Gesamtstrom von 800-850g/h ein. Der Permeatstrom (G) aus der ersten Membranstufe wurde in den Vorlagebehälter(M3) der zweiten Membranstufe gefahren. Dieser Vorlagebehälter (M3) wurde ebenfalls analog zum ersten Vorlagebehälter (M1) mit Synthesegasdruck abgedeckt. Auf den Vorlagebehälter (M3) wurde eine Synthesegasmenge von 40nl/h gegeben.

Die flüssige Phase aus der Vorlage (M3) wurde ebenfalls standgeregelt in die zweite Membranstufe gefahren. Die zweite Membranstufe bestand aus der Druckerhöhungspumpe (P3), der Überströmpumpe (P4), dem Wärmeaustauscher (W2) und dem Flachkanalmodul (M4).

Die Überströmung über die Membran wurde mit der Pumpe (P4) auf 300l/h eingestellt.

Die Rückhalte der Abbaukomponenten, Komponente A und Komponente B, wurde durch Probennahme und HPLC-Analytik des Retentatstroms (E) und des Permeatstroms (G) ermittelt. Der Temperaturbereich in der Membranstufe wurde dazu zwischen 20°C und 90°C eingestellt.

Der Retentatstrom (F) der zweiten Membranstufe wurde wieder zum Vorlagebehälter (M1) der ersten Membranstufe zurückgefahren. Das Permeat (H) der zweiten Stufe wurde in die, ebenfalls synthesegasabgedeckte Vorlage (M5) gefahren. Aus dieser Vorlage(M5) wurde die flüssige Phase (D) aus dem System ausgeschleust. Dieser Strom betrug zwischen 50 und 100g/h.

Als Membranen wurde der PDMS- / Polydimethylsiloxan-basierte Typ oNF2 von GMT eingesetzt.

Die Nanofiltration wurde so betrieben, dass sich die Abbauprodukte des Liganden 5 b, kurz Biphephos oder (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine), Komponente A und Komponente B, nicht im System akkumulieren konnten.

Die Umsatzbestimmung erfolgte mittels eines Gasphasen-GC im Eingangsstrom des Kondensator (B2).

In jedem Reaktor (A1 bis A3) wurde mit Hilfe eines HPLC der Anteil des Liganden bestimmt und dieser durch das Nachfahren einer 2%igen Ligandenlösung in entgastem Rohprodukt (15) auf einem konstanten Niveau gehalten. Diese Lösung enthielt neben dem Liganden 5 b, kurz Biphephos oder (6,6'-((3,3'-di-tert-butyl-5,5'-dimethoxy-[1,1'-biphenyl]-2,2'-diyl)bis(oxy))-bis-dibenzo[d,f][1,3,2]dioxaphosphepine) noch Bis (2,2,6,6-Tetramethyl-4-Piperidinyl) sebacate im molaren Verhältnis 2:1 Bis (2,2,6,6-Tetramethyl-4-Piperidinyl) sebacate zum Liganden Biphephos (6,6'-((3,3'-di-tert-butyl-5,5'-dimethoxy-[1,1'-biphenyl]-2,2'-diyl)bis(oxy))didibenzo[d,f][1,3,2]dioxaphosphepine).

Neben dem Ligandengehalt wurde auch die Viskosität der Reaktionslösung in den Reaktoren (A1-A3) gemessen.

Ein Standanstieg wurde in den Reaktoren konnte auch nach 3000h entsprechend 125 d nicht festgestellt werden und die Viskosität der Reaktionslösung betrug, wie im Beispiel XII < 10 mPas; s. Figur 8.

**Bezugszeichenliste**

(1) Eingang olefinische Verbindung
(2) Synthesegaseingang
(3) Abgas(Purgegas)
(4) Permeat Membranfiltration
(5) Rohprodukt
(6) Retentat Membranfiltration
(7) Rohprodukt angereichertes Kreisgas
(8) Ausgang Reaktion
(9) Kreisgaseingang
(10) Gaspendelleitung
(11) Rohprodukt reduziertes Kreisgas

(12) Reaktorüberlauf
(13) Feed zur Membrananlage
(14) Druckhaltung
(15) Liganden oder Katalysatorlösung
(16) Reaktionslösung

(B1) Strippbehälter
(B2) Kondensator
(B3) Phasentrennbehälter
(B4) Verdichter
(C1) Membrananlage

(A1-A3) Reaktoren
(C) Abgas mit Druckhaltung
(M1/M3/M5) Vorratsbehälter
(P1/P3) Druckerhöhungspumpe
(W1/W2) Wärmetauscher
(M2/M4) Flachkanalmodul mit Membran
(E/F) Retentatstrom
(G/H) Permeatstrom
(P2/P4) Überströmpumpe
(A) Zulauf zur Membrananlage
(B) Synthesegasüberlagerung

## Patentansprüche

1. Verfahren zur Steuerung der Viskosität von Reaktionslösungen in der Hydroformylierung von olefinhaltigen Gemischen umfassend die Schritte:

   i) Bereitstellen eines Gemisches enthaltend gesättigte und olefinisch ungesättigte Kohlenwasserstoffe, einer in der Hydroformylierung katalytisch aktiven Zusammensetzung, eines Gasgemisches aus Kohlenmonoxid und Wasserstoff und mindestens eines Lösungsmittels;
   ii) Inkontaktbringen der Edukte in mindestens einer Reaktionszone.;
   iii) Abtrennen der Produkte, wobei ein Gasstrom, enthaltend ein Gemisch aus gesättigten und olefinisch ungesättigten Kohlenwasserstoffen, Kohlenmonoxid sowie Wasserstoff in mindestens eine Reaktionszone eingeleitet wird unter der Maßgabe, dass die Produkte über die Gasphase aus der Reaktionszone geführt werden;
   iv) Kondensieren der über die Gasphase abgetrennten Produkte und zuführen zur weiteren Aufarbeitung;
   v) Leiten des Sumpfstroms aus mindestens einer Reaktionszone auf mindestens eine Membranfiltration, **dadurch gekennzeichnet, dass** die in der Hydroformylierung katalytisch aktive Zusammensetzung über das Retentat zurückgehalten und wieder in die Reaktionszone zurückgeführt sowie Abbauprodukte der in der Hydroformylierung katalytisch aktiven Zusammensetzung über das Permeat entfernt werden.

2. Verfahren nach Anspruch 1, wobei die in der Hydroformylierung katalytisch aktive Zusammensetzung:

   a) mindestens eine Organophosphorverbindung mit dreiwertigem Phosphor;
   b) mindestens ein Metall aus den Gruppen 8 - 10 des Periodensystems der Elemente;
   c) optional eine stabilisierende Komponente umfasst.

3. Verfahren nach Anspruch 2, wobei die Organophosphorverbindung mit dreiwertigem Phosphor ausgewählt ist unter Phosphinen, Phosphiten, Phosphoniten, Phosphiniten, Phosphoramiditen,
   das Metall ausgewählt ist aus der Gruppe 8 des Periodensystems der Elemente, die stabilisierende Komponente ausgewählt ist unter sterisch gehinderte Aminen.

4. Verfahren nach den Ansprüchen 1 - 3, wobei das Metall Rhodium ist, das sterisch gehinderte Amin mindestens eine 2,2,6,6-Tetramethylpiperidin-Einheit umfasst.

5. Verfahren nach einem der Ansprüche 1 - 4, wobei die Abbauprodukte der in der Hydroformylierung katalytisch

aktiven Zusammensetzung, ausgewählt unter Alkoholen, Phenolen, Diolen, insbesondere Biphenolen, Binaphtholen, über das Permeat entfernt werden.

**6.** Verfahren nach Anspruch 5, wobei die Membranfiltration:

1) in einem Temperaturbereich von 20 - 90 °C;
2) bei einem transmembranen Druck im Bereich von 1,0 - 3,0 MPa;
3) bei einer Viskosität von maximal 10 mPas durchgeführt wird.

**7.** Verfahren nach Anspruch 6, wobei das Molekulargewicht der über das Permeat abgetrennten Abbauprodukte der in der Hydroformylierung katalytisch aktiven Zusammensetzung 400 g/mol nicht übersteigt und 20 - 50 % des Molgewichts der verwendeten Organophosphorverbindung beträgt.

**8.** Verfahren nach Anspruch 7, wobei der Rückhalt der Abbauprodukte der in der Hydroformylierung katalytisch aktiven Zusammensetzung im Retentat 80% oder kleiner, besonders bevorzugt 70% oder kleiner, und insbesondere 50% oder kleiner ist.

**9.** Verfahren nach Anspruch 8, wobei die Membranfiltration bei einer Temperatur von 30°C und 3 MPa transmembranen Druck in Toluol im Bereich von 400 g/mol bis 500 g/mol zumindest punktuell einen Rückhalt von 90 % sowie im Bereich von 210 g/mol bis 310 g/mol zumindest punktuell einen Rückhalt von 60 % aufweist.

## Claims

**1.** Method of controlling the viscosity of reaction solutions in the hydroformylation of olefin-containing mixtures, which comprises the steps:

**i)** providing a mixture containing saturated and olefinically unsaturated hydrocarbons, a composition which is catalytically active in hydroformylation, a gas mixture composed of carbon monoxide and hydrogen and at least one solvent;
**ii)** contacting the starting materials in at least one reaction zone;
**iii)** separating off the products, with a gas stream containing a mixture of saturated and olefinically unsaturated hydrocarbons, carbon monoxide and hydrogen being introduced into at least one reaction zone, with the proviso that the products are discharged via the gas phase from the reaction zone;
**iv)** condensing the products which have been separated off via the gas phase and passing them to further work-up;
**v)** passing the bottom stream from at least one reaction zone to at least one membrane filtration, **characterized in that** the composition which is catalytically active in hydroformylation is retained via the retentate and recirculated to the reaction zone and degradation products of the composition which is catalytically active in hydroformylation are removed via the permeate.

**2.** Method according to Claim 1, wherein the composition which is catalytically active in hydroformylation comprises:

a) at least one organophosphorus compound containing trivalent phosphorus;
b) at least one metal of groups 8 - 10 of the Periodic Table of the Elements;
c) optionally a stabilizing component.

**3.** Method according to Claim 2, wherein the organophosphorus compound containing trivalent phosphorus is selected from among phosphines, phosphites, phosphonites, phosphinites, phosphoramidites, the metal is selected from group 8 of the Periodic Table of the Elements and the stabilizing component is selected from among sterically hindered amines.

**4.** Method according to any of Claims 1 - 3, wherein the metal is rhodium and the sterically hindered amine comprises at least one 2,2,6,6-tetramethylpiperidine unit.

**5.** Method according to any of Claims 1 - 4, wherein the degradation products of the composition which is catalytically active in hydroformylation, selected from among alcohols, phenols, diols, in particular biphenols, binaphthols, are removed via the permeate.

6. Method according to Claim 5, wherein the membrane filtration is carried out:

1) in a temperature range of 20 - 90°C;
2) at a transmembrane pressure in the range 1.0 - 3.0 MPa;
3) at a viscosity of not more than 10 mPas.

7. Method according to Claim 6, wherein the molecular weight of the degradation products of the composition which is catalytically active in hydroformylation which are removed via the permeate does not exceed 400 g/mol and is 20 - 50% of the molecular weight of the organophosphorus compound used.

8. Method according to Claim 7, wherein the retention of the degradation products of the composition which is catalytically active in hydroformylation in the retentate is 80% or less, particularly preferably 70% or less and in particular 50% or less.

9. Method according to Claim 8, wherein the membrane filtration has, at least at one point, a retention of 90% at a temperature of 30°C and a transmembrane pressure of 3 MPa in toluene in the range from 400 g/mol to 500 g/mol and, at least at one point, a retention of 60% in the range from 210 g/mol to 310 g/mol.

## Revendications

1. Procédé pour piloter la viscosité de solutions réactionnelles lors de l'hydroformylation de mélanges contenant des oléfines, comprenant les étapes :

i) fourniture d'un mélange contenant des hydrocarbures saturés et à insaturation oléfinique, d'une composition catalytiquement active lors de l'hydroformylation, d'un mélange gazeux de monoxyde de carbone et d'hydrogène, et d'au moins un solvant ;
ii) mise en contact des réactifs dans au moins une zone de réaction ;
iii) séparation des produits, ce à l'occasion de quoi un courant gazeux contenant un mélange d'hydrocarbures saturés ou à insaturation oléfinique, du monoxyde de carbone, ainsi que de l'hydrogène, est introduit dans au moins une zone de réaction, à la condition que les produits soient évacués de la zone de réaction en phase gazeuse ;
iv) condensation des produits séparés en phase gazeuse, et leur envoi à un traitement ultérieur ;
v) envoi du courant de fond, à partir d'au moins une zone de réaction, à au moins une filtration sur membrane, **caractérisé en ce que** la composition catalytiquement active lors de l'hydroformylation est retenue sous forme d'un rétentat, et est renvoyée dans la zone de réaction, et de même les produits de dégradation de la composition catalytiquement active lors de l'hydroformylation sont éliminés par le perméat.

2. Procédé selon la revendication 1, **caractérisé en ce que** la composition catalytiquement active lors de l'hydroformylation comprend :

a) au moins un composé organophosphoré, contenant du phosphore trivalent ;
b) au moins un métal des Groupes 8 à 10 du Système Périodique des Eléments ;
c) en option, un composant stabilisant.

3. Procédé selon la revendication 2, dans lequel le composé organophosphoré, contenant du phosphore trivalent, est choisi parmi les phosphines, les phosphites, les phosphonites, les phosphinites, les phosphoramidites, le métal est choisi dans le Groupe 8 du Système Périodique des Eléments, le composant stabilisant est choisi parmi les amines à empêchement stérique.

4. Procédé selon les revendications 1 à 3, dans lequel le métal est le rhodium, l'amine à empêchement stérique comprend au moins un motif 2,2,6,6-tétraméthylpipéridine.

5. Procédé selon l'une des revendications 1 à 4, dans lequel les produits de dégradation de la composition catalytiquement active lors de l'hydroformylation, choisis parmi les alcools, les phénols, les diols, en particulier les biphénols, les binaphtols, sont éliminés par le perméat.

6. Procédé selon la revendication 5, dans lequel la filtration sur membrane est mise en oeuvre :

1) sur une plage de températures de 20 à 90°C ;
2) pour une pression transmembranaire comprise dans la plage de 1,0 à 3,0 MPa ;
3) en présence d'une viscosité maximale de 10 mPa.s.

**7.** Procédé selon la revendication 6, dans lequel la masse moléculaire des produits de dégradation séparés par le perméat de la composition catalytiquement active lors de l'hydroformylation ne dépasse pas 400 g/mole, et est de 20 à 50 % de la masse moléculaire du composé organophosphoré utilisé.

**8.** Procédé selon la revendication 7, dans lequel la teneur résiduelle du rétentat en les produits de dégradation de la composition catalytiquement active lors de l'hydroformylation est de 80 % ou moins, d'une manière particulièrement préférée de 70 % ou moins et en particulier de 50 % ou moins.

**9.** Procédé selon la revendication 8, dans lequel la filtration sur membrane présente, à une température de 30°C et pour une pression transmembranaire de 3 MPa, dans le toluène dans la plage de 400 g/mole à 500 g/mole, au moins ponctuellement une teneur résiduelle de 90 %, ainsi que, dans la plage de 210 g/mole à 310 g/mole, au moins ponctuellement une teneur résiduelle de 60 %.

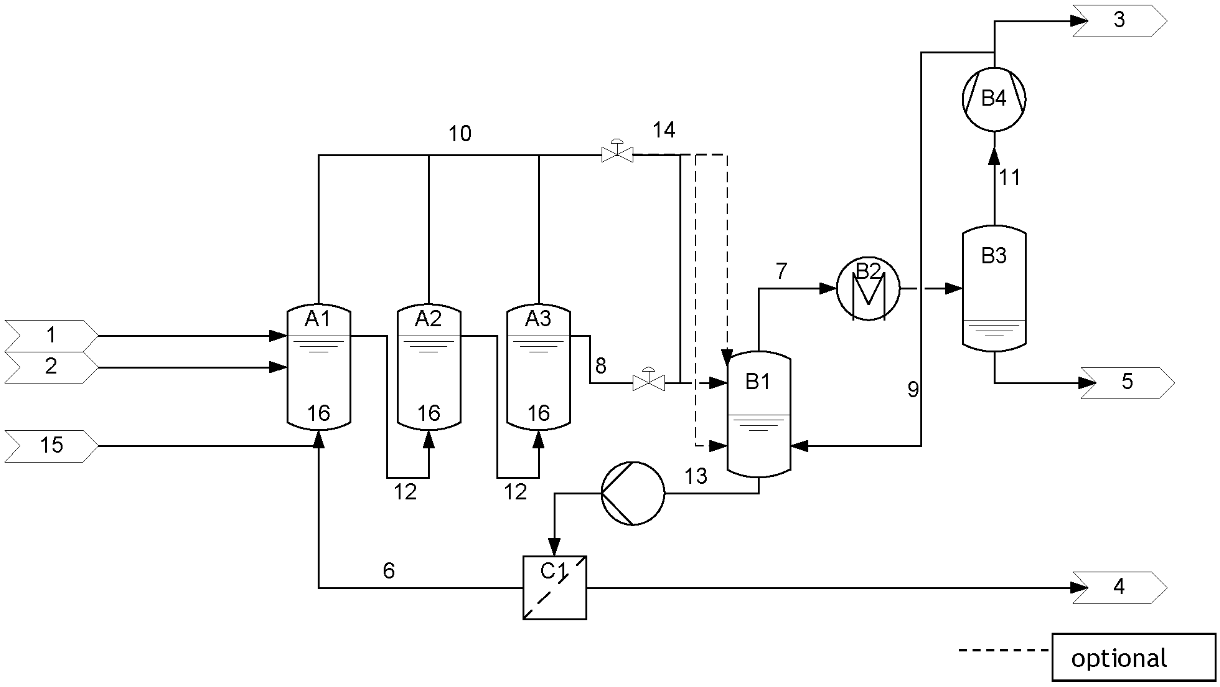
1
2
15
A1
A2
A3
16
16
16
10
14
8
12
12
6
C1
13
B1
7
B2
B3
B4
11
9
3
5
4
optional

Figur 1

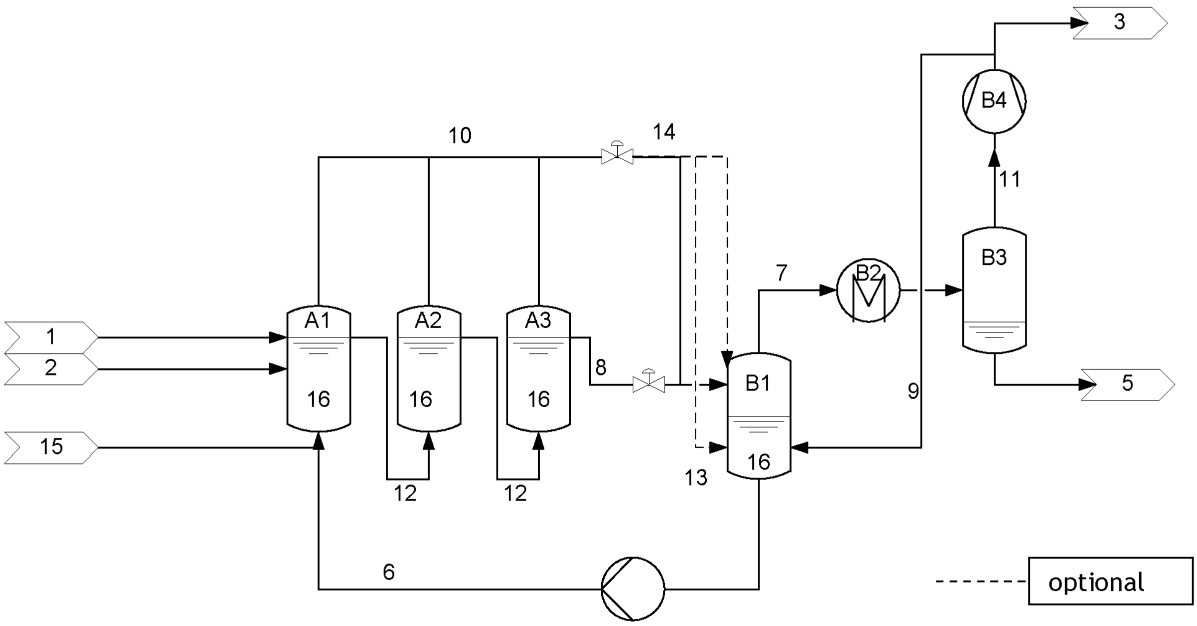

3
B4
10
14
11
B3
7
B2
A1
A2
A3
1
2
8
B1
9
5
16
16
16
15
16
12
12
13
6
optional


Figur 2

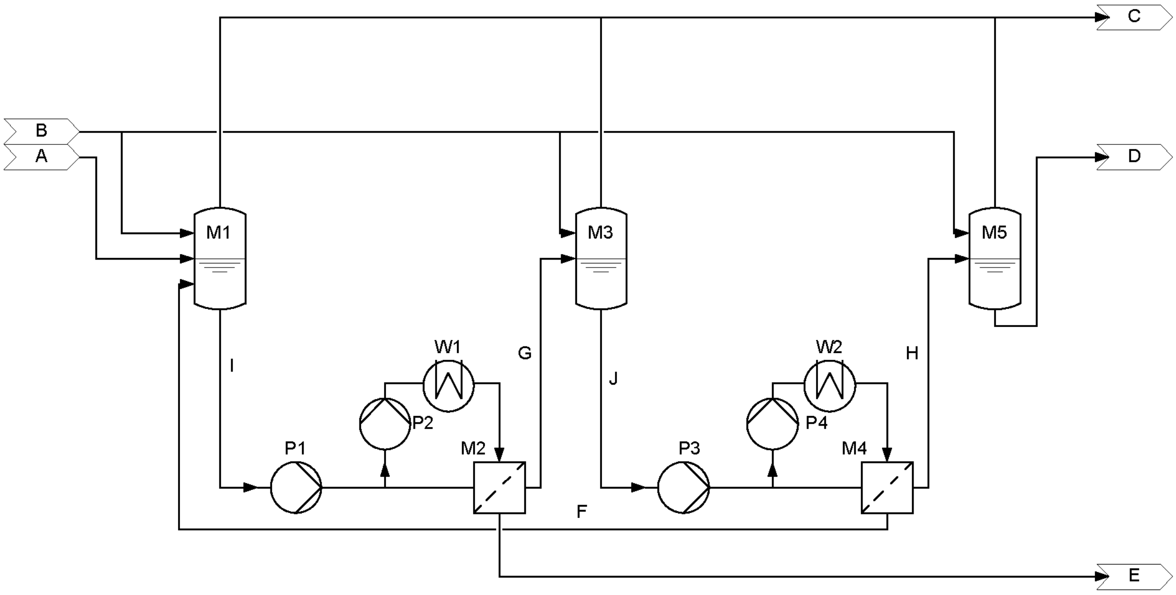


Figur 3

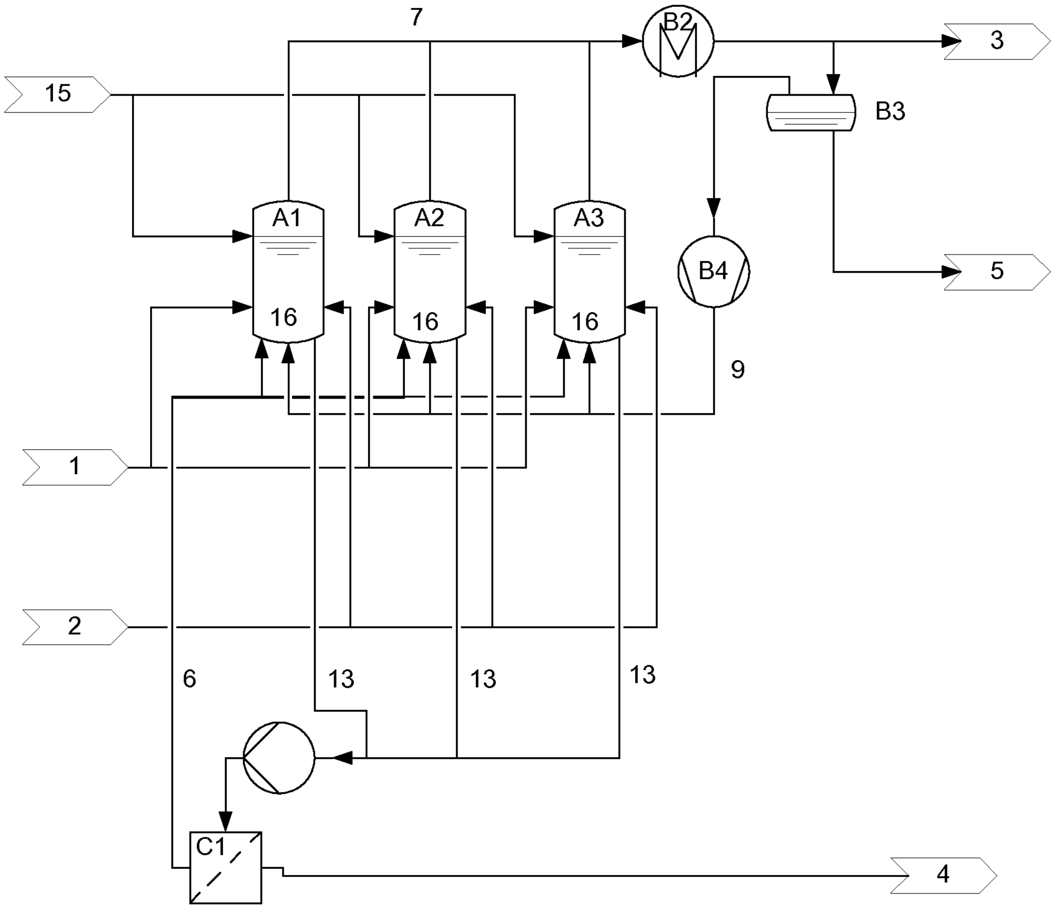


Figur4

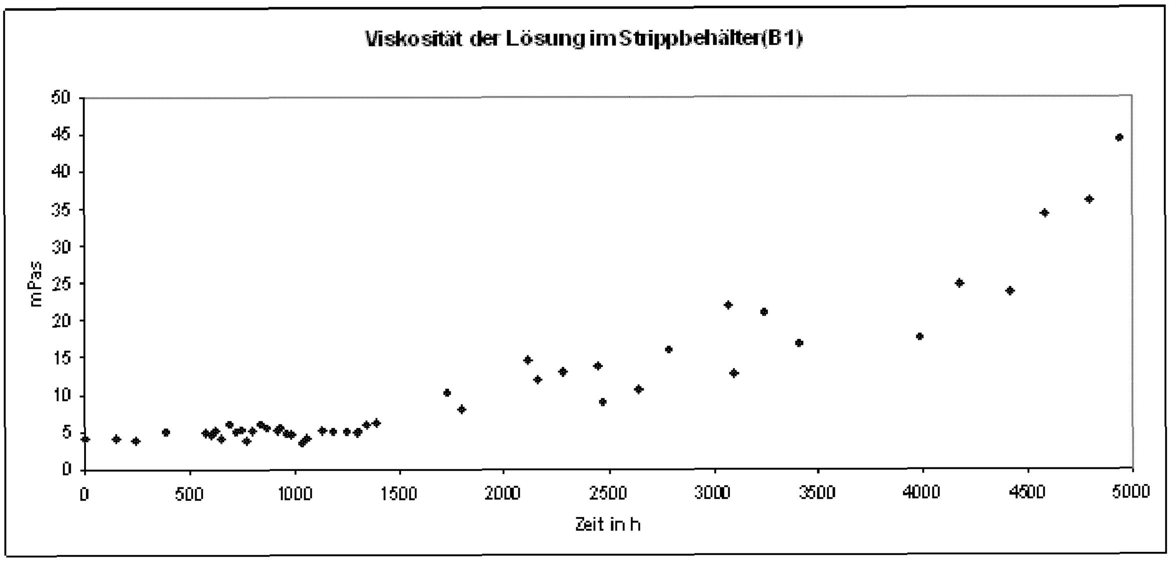
Viskosität der Lösung im Strippbehälter(B1)
mPas
0
5
10
15
20
25
30
35
40
45
50
0
500
1000
1500
2000
2500
3000
3500
4000
4500
5000
Zeit in h

Figur 5

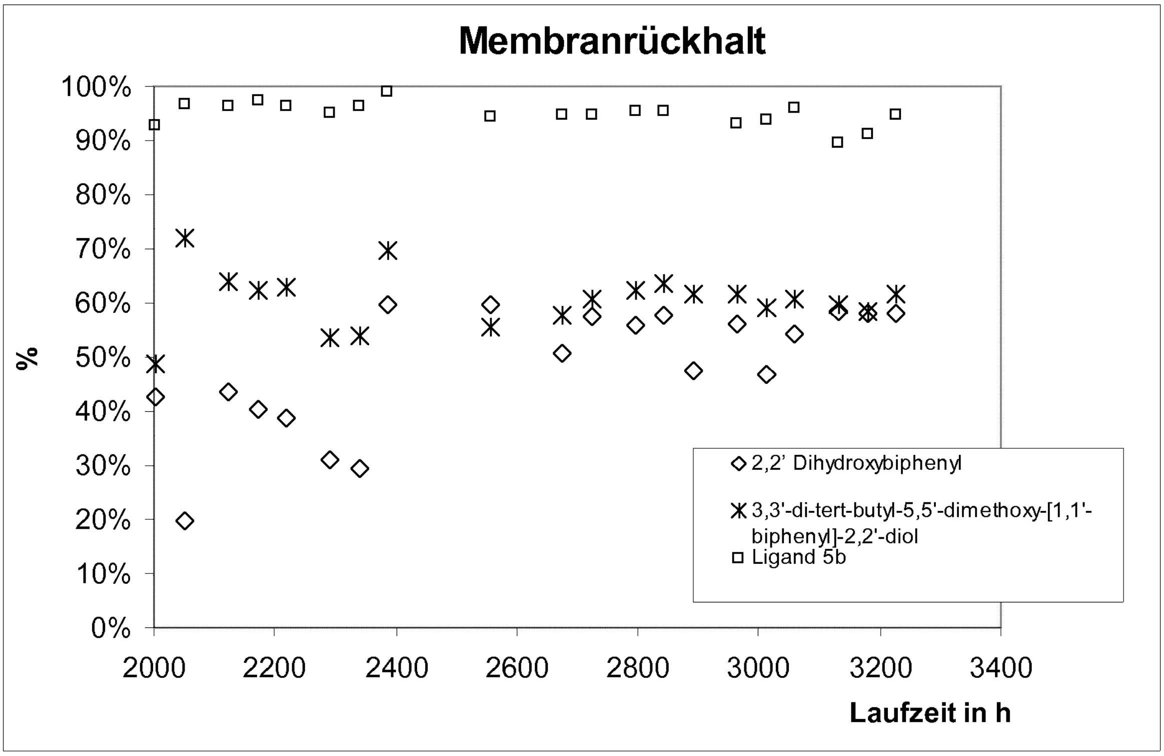
Membranrückhalt
100%
90%
80%
70%
60%
50%
40%
30%
20%
10%
0%
%
2000
2200
2400
2600
2800
3000
3200
3400
Laufzeit in h
2,2' Dihydroxybiphenyl
3,3'-di-tert-butyl-5,5'-dimethoxy-[1,1'-biphenyl]-2,2'-diol
Ligand 5b

Figur 6

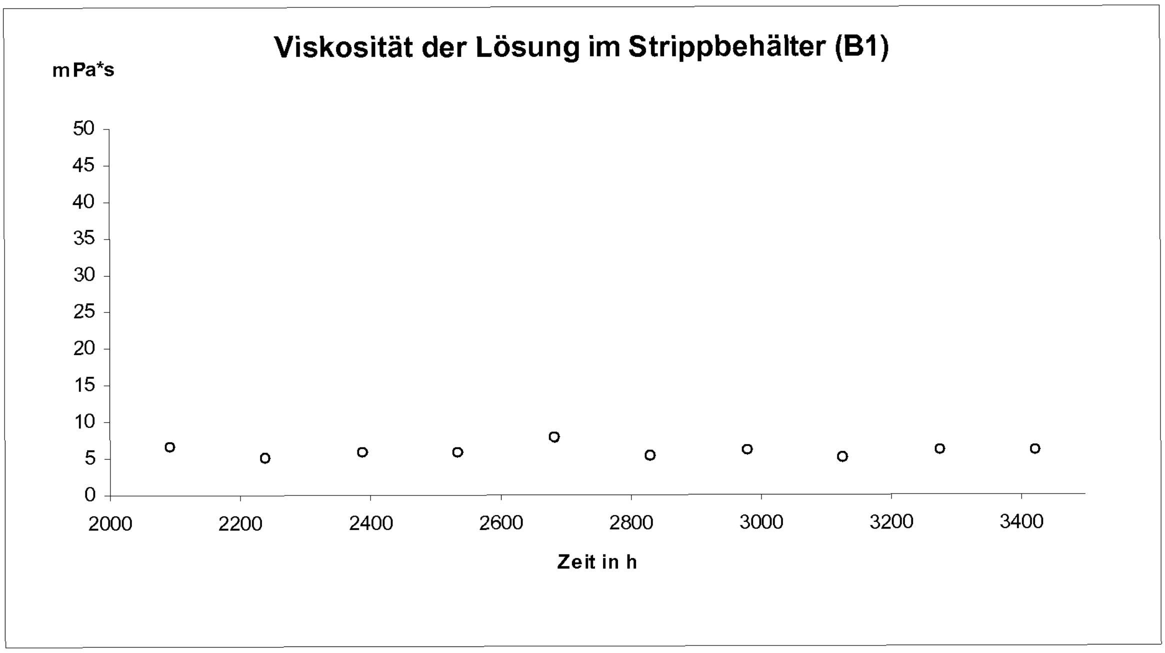
Viskosität der Lösung im Strippbehälter (B1)
mPa*s
50
45
40
35
30
25
20
15
10
5
0
2000
2200
2400
2600
2800
3000
3200
3400
Zeit in h

Figur 7

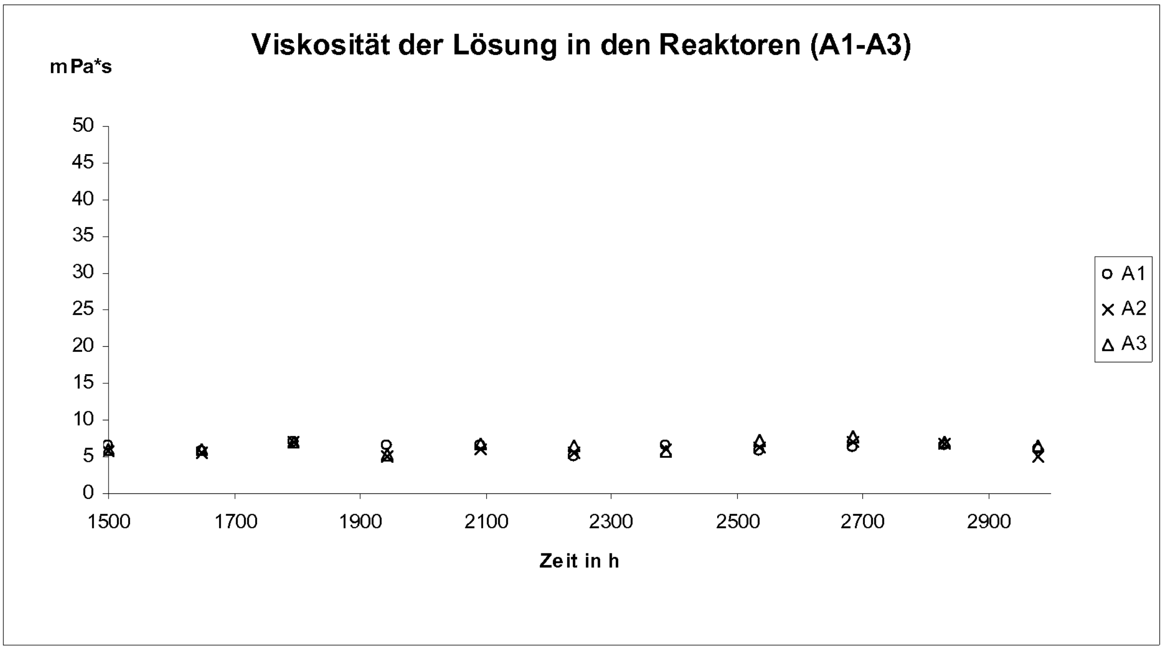
Viskosität der Lösung in den Reaktoren (A1-A3)
mPa*s
50
45
40
35
30
25
20
15
10
5
0
1500
1700
1900
2100
2300
2500
2700
2900
Zeit in h
A1
A2
A3

Figur 8

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102008002187 A1
- WO 2010003073 A
- EP 1931472 B1
- EP 1232008 A
- US 5364950 A
- US 5763677 A
- DE 10308111
- EP 0781166 A
- WO 2010097376 A1
- EP 1430014 B1
- WO 2010003073 A1


### In der Beschreibung aufgeführte Nicht-Patentliteratur


- **B. CORNILS ; W. A. HERRMANN.** Applied Homogeneous Catalysis with Organometallic Compounds. VCH, 1996, vol. 1 & 2
- **R. FRANKE ; D. SELENT ; A. BÖRNER.** Applied Hydroformylation. *Chem. Rev.,* 2012
- Rhodium-catalyzed Hydroformylation. Kluwer Academic Publishers, 2006, 206-211
- Catalyst Separation, Recovery and Recycling. 2006, 25-26
- **PAUL C.J. KAMER ; JOOST N.H.REEK ; PIET W.N.M VAN LEEUWEN.** *Rhodium Catalysed Hydroformylation,* vol. 22, 44
- **D.R. BRYANT.** Catalyst Separation, Recovery and Recycling. Springer, 2006
- **PIET W.N.M. VAN LEEUWEN ; JOHN C. CHADWICK.** Homogeneous Catalysts: Activity - Stability - Deactivation
- **TOH ; X.X. LOH ; A. BISMARCK ; A.G. LIVINGSTON.** In search of a standard method for the characterisation of organic solvent nanofiltration membranes. *J. Membr. Sci,* 2007, vol. 291, 120-125
- Membranes. **I. CABASSO.** Encyclopedia of Polymer Sience and Technlogy. John Wiley and Sons, 1987
- **G. SCHOCK et al.** Mass transfer and pressure loss in spiral wound modules. *Desalination,* 1987, vol. 64, 339-352
- **W. L. F. ARMAREGO ; CHRISTINA CHAI.** Trocknungsmitteln getrocknet (Purification of Laboratory Chemicals. Elsevier, 2009